(19) **Europäisches Patentamt**
**European Patent Office**
**Office européen des brevets**

(11) **EP 2 598 015 B1**

(12) **EUROPEAN PATENT SPECIFICATION**

(45) Date of publication and mention
of the grant of the patent:
**03.09.2014 Bulletin 2014/36**

(51) Int Cl.:
*A61B 1/00* *(2006.01)*     *A61B 1/015* *(2006.01)*
*A61M 25/00* *(2006.01)*     *A61M 25/01* *(2006.01)*
*A61B 1/018* *(2006.01)*

(21) Application number: **11748318.0**

(22) Date of filing: **26.07.2011**

(86) International application number:
**PCT/EP2011/062805**

(87) International publication number:
**WO 2012/013660 (02.02.2012 Gazette 2012/05)**

(54) **ENDOSCOPIC PRESSURE DETECTION ASSEMBLY**

ENDOSKOPISCHE DRUCKERKENNUNGSANORDNUNG

ENSEMBLE DE DÉTECTION DE PRESSION ENDOSCOPIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.07.2010 US 367795 P
26.07.2010 EP 10170794**

(43) Date of publication of application:
**05.06.2013 Bulletin 2013/23**

(73) Proprietor: **Steerable Instruments B.V.B.A.
9900 Eeklo (BE)**

(72) Inventors:
• **DEWAELE, Frank
B-9840 De Pinte (BE)**
• **KALMAR, Alain
B-9000 Gent (BE)**

• **BLANCKAERT, Bart
B-9900 Eeklo (BE)**
• **MABILDE, Cyriel
B-9700 Oudenaarde (BE)**

(74) Representative: **Gyi, Jeffrey Ivan et al
De Clercq & Partners cvba
Edgard Gevaertdreef 10 a
9830 Sint-Martens-Latem (BE)**

(56) References cited:
EP-A1- 1 514 512     WO-A1-2010/023460
DE-A1- 3 206 381     DE-U1- 9 302 891
DE-U1- 20 112 949     DE-U1-202008 010 700
US-A- 5 573 007     US-A1- 2004 024 294
US-A1- 2005 267 417     US-A1- 2009 182 201

**Description**

**FIELD OF THE INVENTION**

The present invention relates to a pressure detection assembly for determining pressure in a tissue cavity inspected by an endoscope, and an endoscope adapted with the assembly, and a method for adapting an endoscope.

**BACKGROUND OF THE INVENTION**

Endoscopy is employed in many surgical procedures such as transurethral resection of the prostate/bladder, hysteroscopic procedures such as endometrial resection, fibroid resection, polyp resection, septoplasty, adhesiolysis and arthroscopic procedures. Increasingly, it is being deployed for neurosurgical procedures, to the extent that endoscopic intraventricular procedures are common in most neurosurgical departments.

In such procedures, a continuous flow endoscope is frequently used. Those skilled in the art would understand the structural composition of a continuous flow endoscope. Briefly, a continuous flow endoscope is adapted to allow rinse fluid simultaneously to enter and escape from a tissue cavity *via* separate entry and exit points, as a result of which positive fluid pressure is created inside the tissue cavity which distends the cavity.

A typical continuous flow irrigation endoscope comprises one or more fluidicly isolated lumens present inside an outer shaft. The outer shaft is typically a hollow cylindrical tube which has a distal end which enters a tissue cavity and a proximal end connected to a hub on which an inflow or outflow port is attached for the purpose of instilling or evacuating fluid from the cavity. The irrigation fluid is instilled *via* an inlet port. The instilled fluid travels through a rinse inlet lumen and enters the tissue cavity *via* the distal opening of the rinse inlet lumen. The waste fluid present inside the tissue cavity enters the distal opening of the rinse outlet lumen, and exits the endoscope via the outflow port attached at the proximal end of rinse outlet lumen.

An optic element (fibre, rigid or chip on tip) is placed inside an inspection lumen of the shaft in order to view the interior of the tissue cavity. An endoscopic instrument, such as a wire loop electrosurgical cutting loop, may be also placed within another lumen present in the shaft.

It was initially assumed that an open outlet channel would prevent a rapid build up of pressure within the tissue cavity. However, in practice, detached tissue pieces, larger than a critical size, present in the tissue cavity are unable to pass through the rinse outlet port leading to obstruction of the outflow channel which can provide an erroneous pressure reading, and pressure-build up. Additionally, the kinking of outflow tube occurs which can also distort the pressure reading. Raised intracranial pressure (ICP) during neurosurgical procedures is common according to the scientific literature, which can induce intracranial hypertension leading to cardiovascular complications, herniation syndrome, retinal bleeding, Terson's syndrome and excessive fluid resorption.

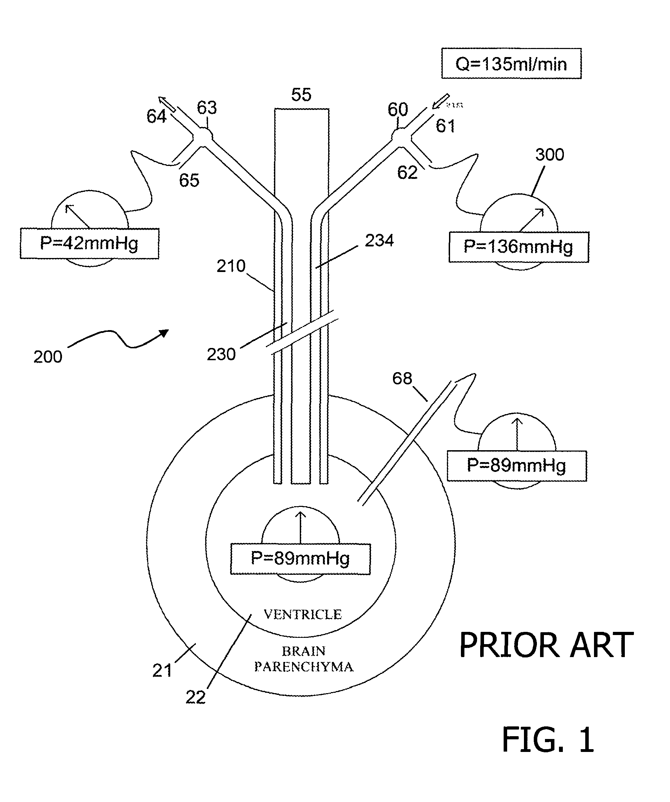

Direct measurement of pressure in the cavity is the gold standard, however, insertion of a separate catheter through a second burr hole for this purpose is clinically impractical and difficult to justify, particularly for the neurosurgeon. Consequently, pressure is presently measured at the proximal end of the endoscope at rinsing inlet and outlet as shown in **FIG. 1** which exemplifies measurement of ICP. However, measurements at the proximal ends of the rinse inlet and outlet have been found to correlate poorly with the actual pressure in the tissue cavity. Adding an extra measuring channel would increase the overall diameter of the endoscope reducing its minimally invasive character.

**FIG. 1** shows an endoscope **200** inserted into the ventricle **22** within the brain parenchyma **21**, provided with a rinse inlet lumen **234** and a rinse outlet lumen **230**. The proximal end **20** of the rinse inlet lumen is connected to a 3-way valve **60**, one branch **62** connected to a pressure gauge **300,** the other branch **61** connected to a pressurized source of irrigation medium. The proximal end **20** of the rinse outlet lumen **230** is connected to a 3-way valve **63**, one branch **65** connected to a pressure gauge, the other branch **64** connected to a waste container. A separate pressure measurement probe **68** is inserted into the ventricle. The true ICP in the ventricle **22** measured by the independent probe **68** is 89 mmHg. A pressure gauge **300** attached to the proximal end of the rinse inlet **62** would indicate a considerably higher pressure (136 mmHg), while a pressure gauge **300** attached to the proximal end of the rinse outlet **65** would indicate a much lower pressure (42 mmHg). From the disparate readings of these proximal gauges, the practitioner must estimate actual ICP, and moreover, be able to determine rapidly blockages to the irrigation circuit.

Pressure measurements at the proximal ends of the inlet and outlet can only provide valid estimations of static ventricular pressures *i.e.* if the rinsing inlet and outlet are closed simultaneously, and pressures are measured after a suitable interval to allow for equilibration of pressures. This is seldom clinically practicable, and it is especially impractical during occasions when high rinsing flows are required such as during brisk bleeding.

Various systems are described in the prior art for the measurement of pressure cavity pressure. For instance US 2009/182201 describes an outer sheath disposed with one or more channels through which a reusable pressure sensor can be inserted, which sheath is fitted over the outer body of an endoscope. The sheath increases the outer diameter of the endoscope, limiting its application in many techniques, including neurosurgery. Moreover, a sterilized sheath is difficult to apply over a sterilized endoscope aseptically. There is thus a need for dynamic pressure assessment that is clinically feasible to implement, which provides a more accurate measurement of actual pressure in the ventricle, can be applied easily aseptically and avoids the problems associated with blockages.

## LEGENDS TO THE FIGURES

**FIG. 1** depicts the prior art configuration for measurement of cavity pressure, showing a cross-section through a continuous flow endoscope and measurement of pressure at the proximal inlet and outlet ports

**FIG. 2** depicts a longitudinal cross-sectional view of pressure detection assembly of the invention that comprises a coupling for dismountable attachment to an endoscope rinse port (inlet or outlet).

**FIG. 3** depicts a longitudinal cross-sectional view of pressure detection assembly of the invention that comprises an alternative coupling for dismountable attachment to an endoscope inlet port (inlet or outlet), which view is marked with dimensions.

**FIG. 3A** depicts a transverse (A-A') cross-sectional view of the elongated member.

**FIG. 4** depicts a cross-sectional view of pressure detection assembly of the invention that comprises a catheter as an elongated member.

**FIG. 4A** depicts a transverse (A-A') cross-sectional view of the elongated member of **FIG. 4.**

**FIG. 5** is a schematic illustration of a fluidic (liquid) pressure gauge.

**FIG. 6** depicts a longitudinal cross-sectional view of pressure detection assembly of the invention where the elongated member comprises a pressure transducer at the tip for measurement of pressure.

**FIG. 6A** depicts a transverse (A-A') cross-sectional view of the elongated member of **FIG. 6.**

**FIG. 7** is a schematic illustration of an electronic read-out unit for a pressure transducer.

**FIG. 8** depicts a longitudinal cross-sectional view of pressure detection assembly of the invention where the elongated member comprises light reflecting element at the tip for the measurement of pressure.

**FIG. 8A** depicts a transverse (A-A') cross-sectional view of the elongated member of **FIG. 8.**

**FIG. 9** is a schematic illustration of a light detection unit for a light-reflecting element.

**FIG. 10** shows a longitudinal cross-sectional view of a continuous flow endoscope.

**FIG. 11** is an enlarged view of an embodiment of the coupling of a pressure detection assembly of the invention.

**FIG. 12** is an enlarged view of an alternative embodiment of the coupling of a pressure detection assembly of the invention.

**FIG. 13** is a longitudinal cross-sectional view of a pressure detection assembly of the invention *in situ* in a continuous flow endoscope.

**FIG. 14** Graph indicating in cavity pressure over time at initiation of rinsing as described in the Example.

**FIG. 15.** Graph indicating in cavity pressure over time during heaving rinsing, measured at the rinsing inlet (I), ventricle drain (V) and rinsing outlet (O) as described in the Example.

**FIG. 16.** Graph indicating in cavity pressure over time, measured at the rinsing inlet (I), ventricle drain (V) and rinsing outlet (O) as described in the Example.

**FIG. 17.** Graph indicating in cavity pressure over time, measured at the rinsing inlet (I), (S), rinsing outlet (O) and using a transendoscopic ventricular tip sensor (S) in the rinse inlet as described in the Example.

**FIG. 18.** Graph indicating in cavity pressure over time, measured at the rinsing inlet (I), (S), rinsing outlet (O) and using a transendoscopic catheter (C) in the rinse inlet as described in the Example.

**FIG. 19.** Graph indicating in cavity pressure over time, measured at the rinsing inlet (I), ventricle drain (V) and rinsing outlet (O) using a short Caemaert endoscope as described in the Example.

## SUMMARY OF THE INVENTION

One embodiment of the invention is a pressure detection assembly (100) adapted for use with a continuous flow endoscope (200) provided with a rinse inlet lumen (234) connected to a rinse inlet port (254) said assembly (100) comprising:

- an elongated member (10) having a proximal (20) and distal (30) end,
- a pressure detecting body (50) at the distal (30) end of the elongated member (10), configured to provide an indication of ambient pressure,
- a coupling (40) disposed over the elongated member (10), configured for dismountable attachment to the proximal rinse inlet port (254) of the endoscope (200),
- said elongated member (10) configured to conduct the indication of ambient pressure to its proximal (20) end,

- said elongated member (10) further configured for advancement through the rinse inlet lumen (234), and
- said fluidic coupling (40) further configured to isolate fluidicly the proximal (20) tip of the elongated member (10) from the rinse inlet port (254) of the endoscope (200).

One embodiment of the invention is a pressure detection assembly (100) adapted for use with a continuous flow endoscope (200) provided with a rinse lumen (232, 234) connected to a rinse port (250, 254) said assembly (100) comprising:

- an elongated member (10) having a proximal (20) and distal (30) end,
- a pressure detecting body (50) at the distal (30) end of the elongated member (10), configured to provide an indication of ambient pressure,
- a coupling (40) disposed over the elongated member (10), configured for dismountable attachment to the proximal rinse port (250, 254) of the endoscope (200),
- said elongated member (10) configured to conduct the indication of ambient pressure to its proximal (20) end,
- said elongated member (10) further configured for advancement through the rinse lumen (232, 234) and to maintain the flow functioning of the rinse lumen, and
- said fluidic coupling (40) further configured to isolate fluidicly the proximal (20) tip of the elongated member (10) from the rinse port (250, 254) of the endoscope (200).

The rinse lumen (232, 234) may be the rinse inlet lumen (234) or the rinse outlet lumen (232), preferably the rinse inlet lumen (234). Accordingly the rinse port (250, 254) may be the rinse inlet port (254) or the rinse outlet port (250), preferably the rinse inlet port (254).

Another embodiment of the invention is an assembly (100) as described above, wherein:

- the elongated member (10) is a single lumen (15) catheter (12), and
- the pressure detecting body (50) comprises the distal (30) tip of the catheter incorporating an open port (14) in fluidic connection with the catheter lumen (15).

Another embodiment of the invention is an assembly (100) as described above, wherein the indication of ambient pressure is conducted by the catheter (12) using hydrostatic force.

Another embodiment of the invention is an assembly (100) as described above, wherein:

- the pressure detecting body (50) comprises an electrical pressure transducer (16), and
- the elongated member (10) comprises a sheath (14) covering electrical wires (17) connecting the transducer (16) to the proximal (20) end of the elongated member (10) for connection to an electronic read-out unit (400).

Another embodiment of the invention is an assembly (100) as described above, wherein the indication of ambient pressure is comprises electrical signals conducted by the wires (17).

Another embodiment of the invention is an assembly (100) as described above, wherein:

- the pressure detecting body (50) comprises a light-reflective element (70) deformable on the application of pressure, and
- the elongated member (10) comprises a sheath (14) covering a plurality of fibre optics (74) connecting the element (70) to the proximal (20) end of the elongated member (10) for connection to an optical detection unit (84).

Another embodiment of the invention is an assembly (100) as described above, wherein the indication of ambient pressure is comprises optical signals conducted by the optical fibres (17).

Another embodiment of the invention is an assembly (100) as described above, wherein the distal tip (30) of the elongated member (10) is configured for locating flush or recessed to the distal (30) tip of the rinse lumen (232, 234). The rinse lumen (232, 234) may be the rinse inlet lumen (234) or the rinse outlet lumen (232), preferably the rinse inlet lumen (234).

Another embodiment of the invention is an assembly (100) as described above, wherein the distal tip (30) of the elongated member (10) is configured for locating at a distance of 0 mm, 0.1 mm, 0.2 mm, 0.4 mm, 0.6 mm, 1 mm, 2 mm, 3 mm, 4 mm, 6 mm, 8 mm, or 10 mm proximal to the distal tip of the rinse lumen (232, 234) or endoscope shaft (220). The rinse lumen (232, 234) may be the rinse inlet lumen (234) or the rinse outlet lumen (232), preferably the rinse inlet lumen (234).

Another embodiment of the invention is an assembly (100) as described above, wherein the outer diameter of the elongated member (10) is adapted to substantially maintain the functioning of the rinse lumen (232, 234). The rinse lumen (232, 234) may be the rinse inlet lumen (234) or the rinse outlet lumen (232), preferably the rinse inlet lumen (234).

Another embodiment of the invention is an assembly (100) as described above, wherein the coupling (40) is configured to separate fluid access to the rinse lumen (234) from fluid access to the catheter (12) lumen (15). The rinse lumen (232, 234) may be the rinse inlet lumen (234) or the rinse outlet lumen (232), preferably the rinse inlet lumen (234).

Another embodiment of the invention is an assembly (100) as described above, wherein the coupling (40) comprises a Luer lock fitting.

Another embodiment of the invention is an assembly (100) as described above, wherein the diameter of the catheter (10, 12) is between 0.3 mm and 0.9 mm, preferably between 0.4 mm and 0.7 mm.

Another embodiment of the invention is an assembly (100) as described above, wherein the length of the catheter (10, 12) between the distal tip and the coupling is between 10 cm and 35 cm, preferably between 14 cm to 24 cm in length.

Another embodiment of the invention is an assembly (100) as described above, wherein the flow functioning of the rinse lumen is maintained when the fluid flow through the rinse lumen (234) at constant pressure is reduced by an amount equal to or less than 60 % when the elongated member (10) is advanced therethrough.

Another embodiment of the invention is a kit comprising:

- an assembly (100) as defined in any of the previous claims, and
- a continuous flow endoscope (200).

Another embodiment of the invention is a method for adapting a continuous flow endoscope (200) with a rinse lumen (232, 234) connected to a rinse inlet port (250, 254) to provide an ambient pressure measurement capability comprising the steps:

(a) providing a pressure detection assembly (100) as defined in any of claims 1 to 9,
(b) advancing the proximal end of the pressure detection assembly (100) through the rinse port (250, 254) of the endoscope 200, and
(c) engaging the coupling (40) with the rinse port (250, 254), thereby adapting the continuous flow endoscope (200) to provide an ambient pressure measurement capability.

The rinse lumen (232, 234) may be the rinse inlet lumen (234) or the rinse outlet lumen (232), preferably the rinse inlet lumen (234). Accordingly the rinse port (250, 254) may be the rinse inlet port (254) or the rinse outlet port (250), preferably the rinse inlet port (254).

Another embodiment of the invention is a method of preparing an assembly as defined above, comprising the step of attaching the fluidic coupling (40) to the shaft of the catheter (12) so as to allow separate fluid access to the rinse lumen (232, 234) and the catheter (12) lumen (15). The rinse lumen (232, 234) may be the rinse inlet lumen (234) or the rinse outlet lumen (232), preferably the rinse inlet lumen (234).

Another embodiment of the invention is a continuous flow endoscope (200) having an outer shaft that encloses a plurality of lumens, further comprising:

- an elongated member (10) having a proximal (20) and distal (30) end, disposed within the shaft,
- a pressure detecting body (50) at the distal (30) end of the elongated member (10), configured to provide an indication of ambient pressure,
- said elongated member (10) configured to conduct the indication of ambient pressure to its proximal (20) end along the elongated member.

Another embodiment of the invention is a continuous flow endoscope (200) as described above, where the elongated member (10) has one or more of the features defined above.

Another embodiment of the invention is a continuous flow endoscope (200) as described above that is single use.

## DETAILED DESCRIPTION OF THE INVENTION

Before the present device and method of the invention are described, it is to be understood that this invention is not limited to particular devices and methods or combinations described, since such devices and methods and combinations may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "having", "including", "includes" or "containing", "contains", and are inclusive or openended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

Whereas the terms "one or more" or "at least one", such as one or more or at least one member(s) of a group of members, is clear *per se,* by means of further exemplification, the term encompasses *interalia* a reference to any one of said members, or to any two or more of said members, such as, *e.g.*, any ≥3, ≥4, ≥5, ≥6 or ≥7 etc. of said members, and up to all said members.

Unless defined otherwise, all technical and scientific terms used herein have the same meaning as is commonly understood by one of skill in the art. All publications referenced herein are incorporated by reference thereto. All United States patents and patent applications referenced herein are incorporated by reference herein in their entirety including the drawings.

The articles "a" and "an" are used herein to refer to one or to more than one, i.e. to at least one of the grammatical object of the article. By way of example, "a port" means one port or more than one port.
The recitation of numerical ranges by endpoints includes all integer numbers and, where appropriate, fractions subsumed within that range (*e.g.* 1 to 5 can include 1, 2, 3, 4 when referring to, for example, a number of articles, and can also include 1.5, 2, 2.75 and 3.80, when referring to, for example, measurements). The recitation of end points also includes the end point values themselves (*e.g.* from 1.0 to 5.0 includes both 1.0 and 5.0).

The terms "distal", "distal end", "proximal" and "distal end" are used through the specification, and are terms generally understood in the field to mean towards (proximal) or away (distal) from the surgeon side of the apparatus. Thus, "proximal (end)" means towards the surgeon side and, therefore, away from the patient side. Conversely, "distal (end)" means towards the patient side and, therefore, away from the surgeon side. Herein, the "proximal (end)" of an element is denoted with reference sign **20**, and the "distal (end)" of an element is denoted with reference sign **30.**

In the passages herein, different embodiments or aspects of the invention are defined in more detail. Each embodiment and/or aspect so defined may be combined with any other aspect or aspects or embodiments unless clearly indicated to the contrary. In particular, any feature indicated as being preferred or advantageous may be combined with any other feature or features indicated as being preferred or advantageous.

In the following detailed description of the invention, reference is made to the accompanying drawings that form a part hereof, and in which are shown by way of illustration only of specific embodiments in which the invention may be practiced. It is to be understood that other embodiments may be utilised and structural or logical changes may be made without departing from the scope of the present invention. The following detailed description, therefore, is not to be taken in a limiting sense, and the scope of the present invention is defined by the appended claims.

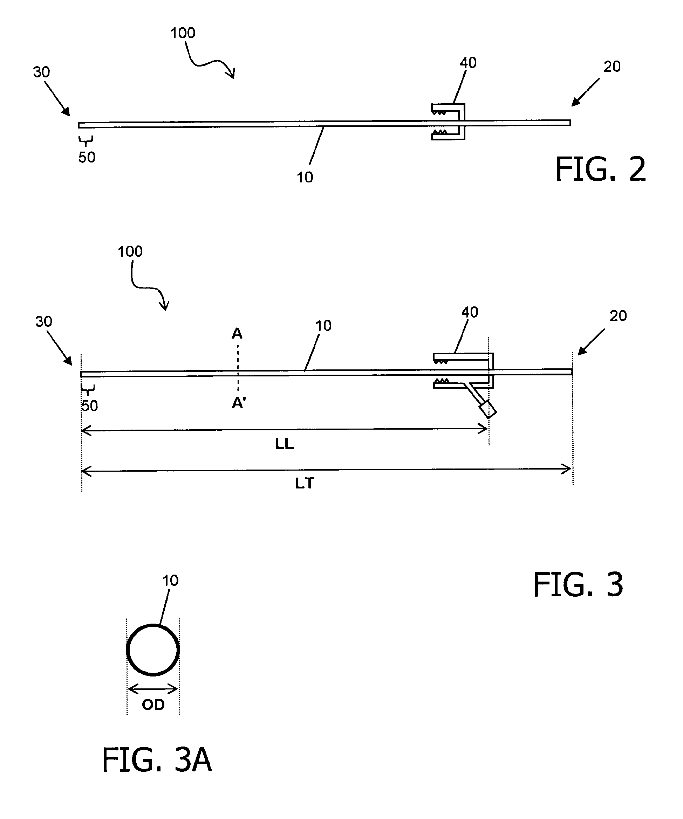

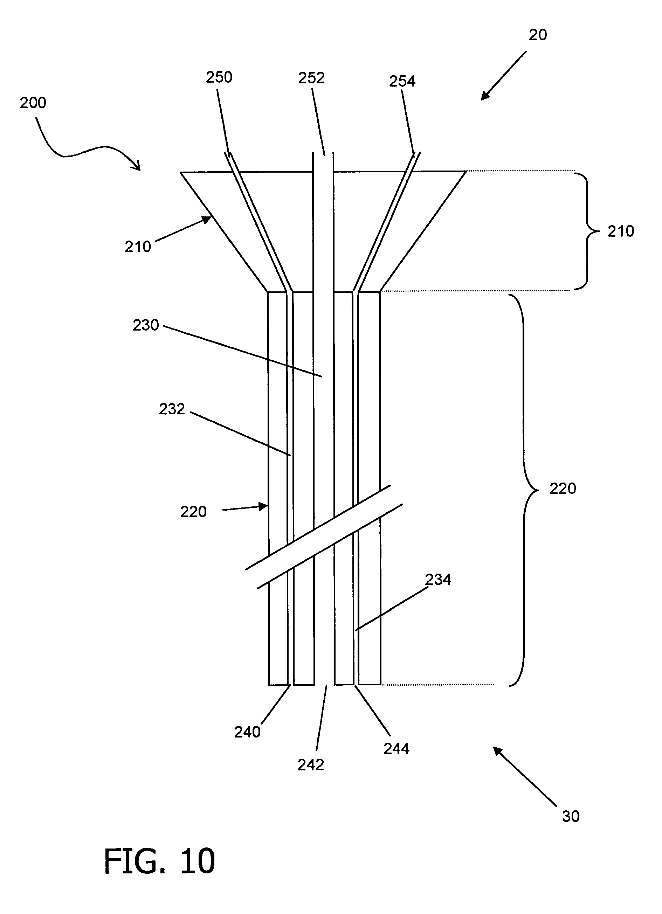

The present invention, as exemplified in **FIG. 2** concerns a pressure detection assembly **100** for use with an endoscope, in particular a continuous flow endoscope, comprising elongated member **10** having a proximal **20** and distal **30** end, disposed at the distal **30** end with a pressure detecting body **50**. The elongated member **10** is provided with a coupling **40** proximal **20** to the pressure detecting body **50** configured for dismountable coupling with a proximal rinse port **250, 254** of an endoscope. Throughout the description, a rinse port **250, 254** is mentioned for coupling with a coupling **40** of the elongated member **10;** this may be the rinse inlet port **254** or the rinse outlet port **250,** but is preferably the rinse inlet port **254.** The coupling **40** provides a water impermeable seal. The elongated tubular member **10** is further configured for advancement into the rinse lumen **232, 234** (**FIG. 10**) of an endoscope **200** towards the distal **30** tip. Throughout the description, a rinse lumen **232, 234** is mentioned through which the elongated member **10** is advanced; this may be the rinse inlet lumen **234** or the rinse outlet lumen **232**, but is preferably the rinse inlet lumen **234.** The outer diameter, **OD**, (**FIG. 3A**) of the elongated tubular member **10** is adapted to permit passage of rinse medium through the rinse lumen **232, 234** when the assembly **100** mounted *in situ i.e.* without substantial hindrance to the flow.

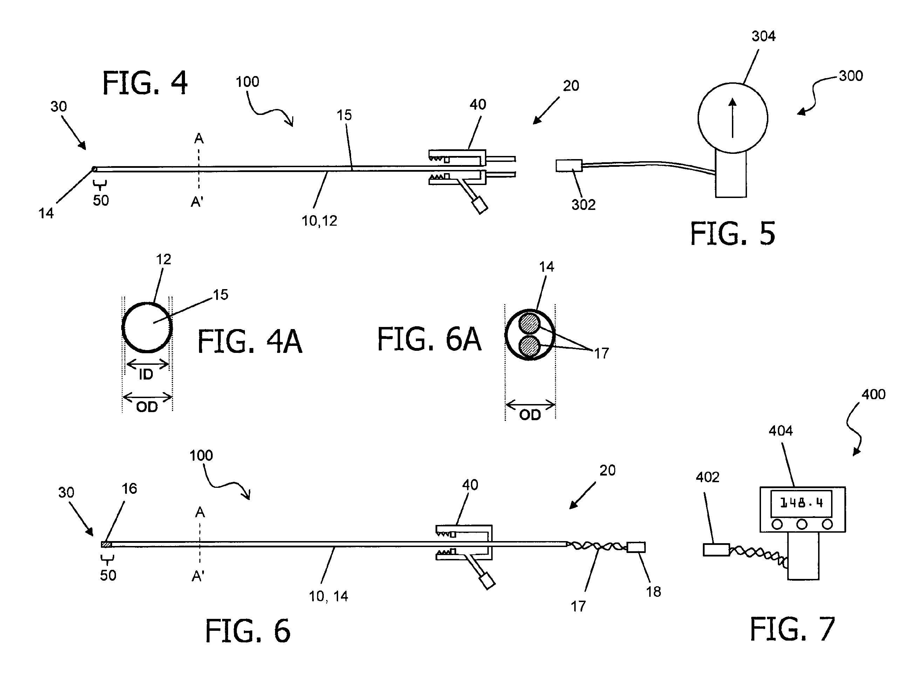

According to one embodiment of the invention, as exemplified in **FIG. 4**, the elongated member **10** comprises a narrow flexible catheter **12** provided with a catheter lumen **15** for conductance of fluid. The pressure detecting body **50** of the catheter **12** comprises an open port **14** at the distal end of the lumen **15**. In normal use, the lumen **15** is filled with a non-compressible fluid for example, a liquid such as aqueous saline solution. Hydrostatic pressure in the vicinity of the pressure detecting body **50** is conducted along the lumen **15** by the non-compressible fluid to the open proximal **20** end of the catheter **12** where a pressure gauge **300 (FIG. 5)** in fluidic connection with the lumen **15** measures the pressure transmitted from the distal end of the catheter. Absolute (or gauge) pressure in the vicinity of the pressure detecting body **50** can thus be recorded. **FIG. 5** depicts a pressure gauge **300** provided with a connector **302** for attachment to the coupling **40** of the catheter.

According to another embodiment of the invention, as exemplified in **FIG. 6,** the pressure detecting body **50** comprises an electrical pressure transducer **16**. Such pressure transducers, known in the art, generate an electrical signal responsive to pressure or pressure changes in the environment of the transducer. They can provide a measure of absolute (or gauge) pressure. Electrical wires **17** connect the transducer **16** to an electronic read-out unit **400 (FIG.**

**7),** optionally via a reciprocating pair of dismountable electrical connectors **18, 402,** which wires are conveyed from the transducer **16** to the proximal end **20** of the assembly **100** through the elongated member **10.** Absolute (or gauge) pressure in the vicinity of the pressure detecting body **50** can be recorded by the electrical read-out unit **400. FIG. 7** depicts an electrical read-out unit **400** provided with a dismountable electrical connector **402** for attachment to a reciprocating connector **18** on the assembly **100.**

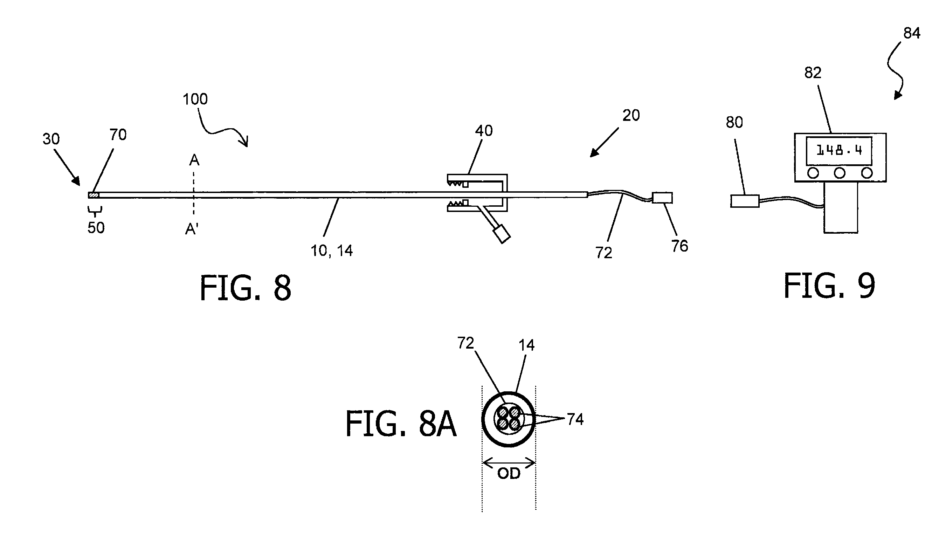

According to another embodiment of the invention, as exemplified in **FIG. 8,** the pressure detecting body **50** comprises a light-reflective element (LRE) that is deformable on the application of pressure. Light arriving at the LRE, through a fibre optic in the elongated tubular member is reflected by the LRE responsive to ambient pressure, and returned back along the elongated tubular member via a separate fibre optic. Optical fibres connect the LRE to an optical detection unit, optionally via a dismountable connector, at the proximal end **20** of the assembly **100.** Distortions of the light-reflective element may cause a phase shift which is then measured in the optical detection unit. The incident light may be supplied by a laser light source. **FIG. 9** depicts an optical detection unit **84** provided with a dismountable optical connector **80** for attachment to a reciprocating connector **76** on the assembly **100.**

Advantageously, the assembly provides a measurement of ambient pressure at the distal end of the endoscope that accurately reflects the static pressure in the cavity of the intervention. By contrast, measurement at the rinsing inlet gives a severe overestimation of the true cavity pressure, and if clinicians were to respond to these pressures, this would unnecessarily impede the rinsing efforts of the surgeon. Measurement at the rinsing outlet gives a systematic severe underestimation of the true cavity pressure, which would delay crucial intervention. Pressure could be measured in a static mode, however, this would require pausing the flow of rinsing fluid. Since rinsing is essential to give the surgeon an unobscured view of the cavity, regular pausing impedes progress and increases operating times, so increasing the costs of interventions and the risk of infections. By employing a pressure detection assembly of the invention, which can be applied to an existing endoscope, the pressure measurement, which accurately reflects pressure in the cavity, can be measured accurately and reliably while the endoscope is rinsing i.e. fluid is flowing through the rinse inlet and rinse outlet. Since the invention can be applied to an existing endoscope, and utilise existing hydrodynamic measurement equipment when the elongated member **10** is a catheter **14,** the costs of implementation are neglible.

Moreover, pressure in the cavity can be exquisitely controlled i.e. set to a pre-defined level and maintained at that level. This can be achieved using a closed-loop (feedback loop) system comprising one or more pumps and/or valves and a controller, which controller regulates said pumps and/or valves responsive to pressure measured using the pressure detection assembly of the invention **100,** to maintain pressure at the predefined level. The closed loop system may be advantageously employed to deliberately increase pressure at the intervention site, which pressure increase expedites clot formation in the case of a hemorrhage. Controllably increasing pressure at the intervention site, particularly in the brain, is presently considered a risk to the extent that many surgeons will avoid it. Since the pressure detection assembly of the invention **100** provides such an accurate reading, procedures otherwise excluded as entailing too much risk, now become available through the present invention. Since an existing endoscope can be adapted, and existing pressure detection equipment employed, the costs of making new pressure-reliant techniques available is negligible.

When the elongated member **10** is a catheter **14,** the costs of implementation are neglible. Moreover, the catheter **14** can be made a disposable item, while the endoscope is sterilized between uses. Since the catheter is provided as a removable and disposable item, problems with steam sterilization are avoided which arise from the lack of steam penetration within the catheter lumen. While steam sterilisation does not affect the endoscope as it is disposed with wider bore lumens, it would not be possible to sterilize the lumen of a catheter **14** inserted into the endoscope channel owing to its narrow diameter. Thus the disposable pressure detection assembly **100** of the invention overcomes this problem.

Advantageously, when the rinse inlet channel **234** is used, fresh rinse medium continually washes over the pressure detecting body, removing or preventing blockages or contamination with particles. The assembly **100** provides an economical solution to the problem, that can be deployed on existing endoscopes without significant adaptation.

The assembly **100** of the invention is configured for connection with the rinse lumen **232, 234** of an endoscope **200.** The rinse lumen **232, 234** may be the rinse inlet lumen **234** or the rinse outlet lumen **232,** preferably the rinse inlet lumen **234.** The endoscope is preferably a continuous rinse endoscope. As explained elsewhere herein, a continuous flow endoscope is adapted to allow fluid simultaneously to enter and escape from a tissue cavity via separate entry and exit points. It is well known to the person skilled in the art. As a general guidance and with reference to **FIG. 10,** a continuous flow endoscope **200** comprises a rigid or flexible shaft **220** that encloses a plurality of lumens **230, 232, 234** in fluidic isolation from each other within the shaft, which lumens **230, 232, 234** span the length of the shaft **220.** Typically, there is an inspection lumen **232** for receiving a visualisation instrument (e.g. camera, fiber optic or rigid optic), and two rinse lumens **232, 234** and one or two working channels to insert instruments. One or more additional lumens may be present, for example, to receive a medical instrument. As mentioned earlier, a rinse inlet lumen **234** allows passage of fresh rinse medium to the distal **30** tip of the endoscope, while a rinse outlet lumen **232** provides a passage for removal of waste rinse medium from the intervention site. The rinse medium employed is dependent on the tissue cavity under investigation, but will generally contain a buffered aqueous saline solution.

Each lumen **230, 232, 234** is open at the distal **30** end, the open end provided with an exit port **240, 242, 244.** Said exit ports **240, 242, 244** are typically disposed as a bundle within the shaft **220** at the distal **30** tip. The proximal end **20** of the shaft **210** is attached to a hub **210.** The hub **210** comprises a plurality of proximal coupling ports **250, 252, 254;** each lumen **232, 230, 234** being in fluidic connection with one or more proximal coupling ports **250, 252, 254.** In particular, the inspection lumen **230** is in fluidic connection with an inspection port **252,** the rinse inlet lumen **234** is in fluidic connection with a rinse inlet port **254,** and the rinse outlet lumen **232** is in fluidic connection with a rinse outlet port **250.** The hub allows the dismountable attachment of a connector, valve, gauge etc to each port for servicing the requisite lumen. To this end, each port **250, 252, 254** may be provided with a connector such as a male or female screw thread connector. In **FIG. 10,** each port is shown with a male connector. A continuous flow endoscope is a standard piece of surgical equipment, of known configuration and available in a standard size.

The elongated member **10** of the assembly **100** is configured for advancement through a rinse lumen **232, 234.** The rinse lumen **232, 234** may be the rinse inlet lumen **234** or the rinse outlet lumen **232,** preferably the rinse inlet lumen **234.** As such, the elongated member **10** is flexible, but stiffened to provide pushability through the lumen **232, 234** without kinking or buckling. Typically it has a cylindrical transverse sectional profile, though other profiles are not excluded such as ovoid or polygonal. The elongated member **10** of the assembly **100** is further configured to conduct an indication of ambient pressure generated by the pressure detecting body **50** to its proximal **20** end. The type of conductance is dependent on the pressure detecting body **50.** When pressure detecting body **50** comprises a port **14 (FIG. 4),** hydrostatic forces generated *via* the port are conducted by the elongated member **10** by virtue of non-compressible fluid therein. When pressure detecting body **50** comprises a pressure transducer **16 (FIG. 6),** electrical signals generated are conducted by wires in the elongated member. When pressure detecting body **50** comprises an LRE **(FIG. 8),** optical signals generated are conducted by fibre optics in the elongated member. The pressure indication at the proximal end is read by a pressure gauge (*e.g.* electronic or mechanical) where there are hydrostatic force fluctuations, or by an electronic read-out unit when the electrical signals are generated or by an optical detection unit when the light signals are generated.

The length of the elongated member **10** is such that the pressure detecting body **50** is able to advance through the rinse lumen **232, 234** towards the distal **30** tip of the endoscope **100.** Preferably, the length is such that the pressure detecting body **50** is in a recessed or flush position relative to the distal **30** tip of the rinse lumen **232, 234** or the endoscope shaft **10,** particularly when the coupling **40** of the assembly **100** is connected to the rinse port **250, 254** of the endoscope **200.** While a recessed or flush juxtaposition is preferred, it is within the scope of the invention that the length of the elongated member **10** allows it to protrude relative to the distal **30** tip of the rinse lumen **232, 234** or the endoscope shaft **10,** for example, by 1, 2 or 3 mm. It will be appreciated that the length of the protrusion is minimised to avoid a risk of damaging the tissue under intervention.

The luminal length, **LL (FIG. 3)** of elongated member **10,** which is the distance between the coupling **40** and the distal **30** tip of the pressure detecting body **50.** More precisely, it is the length of the elongated member **10** from the distal **30** tip of the pressure detecting body **50** to where it joins to the coupling **40.** The luminal length, **LL** is preferably equal to or less than the length of the rinse lumen **232, 234.** The luminal length, **LL** may be adjustable, for instance, by employing a coupling **40** in sliding relation to the elongated member **10,** or by truncation of the elongated member **10** from the distal end when it is formed from a catheter **12.**

As a general guidance, the luminal length, **LL (FIG. 3)** of elongated member **10** between the coupling **40** and the distal **30** tip of the pressure detecting body **50** may be equal to or less than 99 %, 98 %, 96 %, 94 %, 92 %, 90 %, 85 %, 80 %, 75 % of the length of the rinse lumen **232, 234** (*i.e.* the rinse inlet lumen **234** or the rinse outlet lumen **232),** or a value between any two of the aforementioned values. *In situ* coupled to the endoscope **200,** pressure detecting body **50** is in a flush or recessed, but not protruding configuration relative to the distal **30** tip of the rinse lumen **232, 234** or endoscope shaft **10.** Preferably distal tip of the pressure detecting body **50** is at a distance of 0 mm, 0.1 mm, 0.2 mm, 0.4 mm, 0.6 mm, 1 mm, 2 mm, 3 mm, 4 mm, 6 mm, 8 mm, or 10 mm proximal to the distal tip of the rinse lumen **232, 234** or endoscope shaft **220.** According to one aspect of the invention, the luminal length, **LL** of the elongated member **10** is equal to or less than 10 cm, 12 cm, 14 cm, 16 cm, 18 cm, 20 cm, 22 cm, 24 cm, 26 cm, 28 cm, 30 cm, 32 cm, 34 cm, 35 cm, 36 cm, 38 cm, 40 cm or is a value in the range between any two of the afore mentioned values.

The maximum outer diameter **OD (FIG. 3A)** of the elongated member **10** is smaller than the inner diameter of the rinse lumen **232, 234** (*i.e.* of the rinse inlet lumen **234** or the rinse outlet lumen **232**).

Additionally, it is configured to substantially maintain the flow functioning, more particularly a proper functioning, of the rinse lumen *i.e.* the flow of rinsing medium through the rinse inlet lumen **234** or through the rinse outlet lumen **232** is not substantially hindered when the elongated member **10** is deployed in the endoscope. According to one embodiment of the invention, the maximum outer diameter **OD (FIG. 3A)** of the elongated member **10** is equal to or less than 5 %, 10 %, 20 %, 30 %, 40 %, 50 %, 60 %, or 70 % of the inner diameter of the rinse inlet lumen **234,** or a value between any two of the aforementioned values, preferably between 5% and 50 %. According to another embodiment of the invention, the maximum outer diameter **OD (FIG. 3A)** of the elongated member **10** is equal to or less than 5 %, 10 %, 20 %, 30 %, 40 %, 50 %, 60 %, or 70 % of the inner diameter of the rinse outlet lumen **232,** or a value between any two of the aforementioned values, preferably between 5% and 50 %. As a general guidance, the **OD** may be 0.3 mm, 0.4 mm, 0.5 mm, 0.6 mm, 0.7 mm, 0.9 mm, 1.1 mm, 1.3 mm, 1.5 mm, or a value in the range between any two of the aforementioned values, preferably 0.4 mm to 0.7 mm, preferably 0.5 mm to 0.7 mm or most preferably 0.6 mm; this is particularly applicable when the inner diameter of the rinse inlet lumen or rinse outlet lumen is 1.67 mm.

Determining whether the flow of rinsing medium through the rinse inlet lumen **234** or through the rinse outlet lumen **232** is not substantially hindered when the elongated member **10** is deployed in the endoscope can be determined by flow rate tests. The fluid flow rate in the rinse lumen **(232, 234)** is measured at constant pressure without and with the elongated member **10** deployed therein. Should the flow rate drop by a certain amount, then the elongated member **10** plays a substantially hindering role. Typically, the flow functioning of the rinse lumen is maintained when the fluid flow at constant pressure is reduced by 0 %, or an amount equal to or less than 5 %, 10 %, 20 %, 30 %, 40 %, 50 %, 60 % when the assembly is coupled to the rinse lumen, more particularly, when the elongated member **10** is deployed therein, compared to when it is absent.

When the pressure detecting body **50** comprises a distal open port **14 (FIG. 4)**, the elongated member **10** may be a single channel catheter **12** provided with a catheter lumen **15** extending between said port **14** and an open proximal **20** end for conductance of a non-compressible fluid, for example, saline solution. As shown in **FIG. 4A** depicting a transverse **(A-A')** cross-section of the catheter **12** shaft, the outer wall of the catheter shaft **12** is complete and intact along the length, to provide a fluid impermeable passage that facilitates transport of fluid along the lumen **15** responsive to changes in pressure at the pressure detecting body **50**. The distal end **30** of the catheter **12** is provided with a distal open port **14** that is in fluidic connection with the open proximal **20** end of the catheter **12**. An example of a suitable catheter is the thin walled polyimide catheter (Microlumen, Tampa, Florida) provided with or without a stainless steel braid . As explained later below, the body or outer wall of the proximal **20** end or portion of the catheter **12** is sealably connected to a coupling **40** for the relevant endoscope rinse port **252, 254,** which coupling **40** is configured to fluidicly isolate the open proximal **20** end of the catheter **12** from the rinse port **252, 254** of the endoscope **200**. The rinse port **250, 254** may be the rinse inlet port **254** or the rinse outlet port **250,** preferably the rinse inlet port **254**. More in particular, it is configured to allow the fluidic connection of the pressure gauge **304** to the open proximal **20** end of the catheter **12** while excluding the fluidic connection of said gauge **304** to the relevant rinse port **252, 254** of the endoscope **200**. Hydrostatic pressure is thereby measured at the proximal **20** end of the catheter **12** lumen **15** to the exclusion of a hydrodynamic measurement at the proximal **20** end of the rinse lumen **232, 234** to which the assembly **100** is coupled.

As a general guidance, maximum outer diameter, OD, of the catheter **12** shaft **(FIG. 4A),** particularly in the region that will pass through the rinse lumen **232, 234** may be equal to or less than 0.3 mm, 0.4 mm, 0.5 mm, 0.6 mm, 0.7 mm, 0.8 mm, 0.9 mm or a value in the range between any two of the aforementioned values, preferably between 0.4 mm and 0.7 mm, 0.5 mm and 0.7 mm, most preferably 0.6 mm; the preferred values are particularly applicable when the inner diameter of the rinse inlet lumen **234** or rinse outlet lumen **232** is 1.67 mm.

The inner diameter, **ID,** of the catheter lumen **15 (FIG. 4A)** is sufficient to allow the passage of non-compressible fluid without substantial hindrance. According to one aspect of the invention, the minimum diameter of the catheter lumen **15** is equal to or no more than 10 %, 20 %, 30 %, 40 %, 50 %, 60 %, 70 %, 75%, 80 %, 90 % of the outer diameter, OD, of the catheter **12** shaft, or a value in the range between any two of the aforementioned values, preferably between 70 % and 80 %, most preferably 75%; the preferred values are particularly applicable when the OD of the catheter shaft is 1.67 mm.

The length of the catheter **12** shaft is such that the distal open port **14** is able to pass through a rinse lumen **232, 234** towards the distal **30** tip of the endoscope **100**. Preferably, the length is such that the distal open port **14** is located in a recessed or flush position relative to the distal **30** tip of the rinse inlet lumen **232, 234** or the endoscope shaft **10,** particularly when the coupling **40** of the assembly **100** is connected to the relevant rinse port **250, 254** of the endoscope **200**.

The dimensions of the luminal length, **LL (FIG. 3)** mentioned above in respect of the elongated member **10** apply equally to the catheter **12**. According to one aspect of the invention, the luminal length, **LL** of the catheter **12** is equal to or less than 10 cm, 12 cm, 14 cm, 15 cm, 16 cm, 18 cm, 20 cm, 22 cm, 24 cm, 26 cm, 28 cm, 30 cm, 32 cm, 34 cm, 35 cm, 36 cm, 38 cm, 40 cm or is a value in the range between any two of the afore mentioned values. Preferably, it is between 15 cm and 40 cm in length.

The luminal length, **LL (FIG. 3)** of the catheter **12** between the coupling **40** and the distal open port **14** is preferably equal to or less than the length of the rinse lumen **232, 234** where it is deployed, *i.e.* of the rinse inlet lumen **234,** or the rinse outlet lumen **232,** preferably the rinse inlet lumen **234**. The luminal length, **LL** may be fixed. Alternatively, the luminal length, **LL** may be adjustable, for instance, by employing the coupling **40** in sliding relation to the elongated member **10**. Alternatively, it may be adjustable by truncation of the elongated member **10** at the distal **30** end; in other words, catheter part **12** of the assembly **100** may be provided at a length that exceeds the length of the relevant endoscope **200** rinse lumen **232, 234,** and the practitioner can trim the distal **30** portion according to requirements.

The wall of the catheter shaft **12** may be formed from any suitable non-expandable material such as polyethylene, polypropylene, or polyimide.

When the pressure detecting body **50** comprises an electrical pressure transducer **16 (FIG. 6)**, the elongated member **10** is a sheath **14** that forms a protective covering for electrical wires **17** connecting the transducer **16** to the electrical read-out unit **400** which is capable of receiving electrical signals, optionally via an electrical connector. The electrical read-out unit **400** is adapted to receive electrical signals from the transducer, to display pressure data therefrom, and/or transfer the pressure data to a computer using, for example, a serial interface. As shown in **FIG. 6A** depicting a transverse **(A-A')** cross section of the sheath **14**, the sheath **14** houses the necessary wires **17,** maintaining them in intimate longitudinal contact so reducing the profile of the elongated member **10**.

As explained later below, the proximal **20** end of the sheath body is in sealed connection with a coupling **40** for an endoscope rinse port **250, 254,** which may be the rinse inlet port **254** or the rinse outlet port **250,** preferably the rinse inlet port **254**. The coupling **40** is configured to seal the proximal **20** region of the sheath **14** body with the selected rinse port **250, 254** of the endoscope **200**. More in particular, it is configured to allow electrical wires **17** connecting the transducer to exit the sheath **14,** while providing a separate fluidic connection to the rinse port **250, 254** of the endoscope **200** hub **210**.

As a general guidance, maximum outer diameter, OD, of the sheath **14 (FIG. 6A),** particularly in the region that will pass through the relevant rinse lumen **232, 234** may be equal to or less than 0.3 mm, 0.4 mm, 0.5 mm, 0.6 mm, 0.7 mm, 0.8 mm, 0.9 mm 1.0 mm, 1.1 mm, 1.2 mm, 1.3 mm, 1.4 mm, 1.5 mm or a value in the range between any two of the aforementioned values, preferably between 0.4 mm and 0.7 mm, 0.5 mm to 0.7 mm, most preferably 0.6 mm; the preferred values are particularly applicable when the inner diameter of the rinse inlet lumen **234** or rinse outlet lumen **232** is 1.67 mm.

The dimensions of the luminal length, **LL (FIG. 3)** mentioned above in respect of the elongated member **10** apply equally to the sheath **14**.

The luminal length, **LL (FIG. 3)** of the sheath **14** between the coupling **40** and the distal open port **14** is preferably equal to or less than the length of the relevant rinse lumen **232, 234**. The luminal length, **LL** may be fixed. Alternatively, the luminal length, **LL** may be adjustable, for instance, by employing the coupling **40** in sliding relation to the sheath **14**.

The wall of the sheath **14** may be formed from any suitable material such as polyethylene or polypropylene, polyimide, polyether ether ketone (PEEK).

When the pressure detecting body **50** comprises a light-reflective element (LRE) **70 (FIG. 8)**, the elongated member **10** is a sheath **14** that forms a protective covering for the optical fibres **74** connecting the LRE **70** to the optical detection unit **84** which is capable of receiving optical signals, optionally via a pair of reciprocating connectors **76, 80**. The optical detection unit **84** is adapted to receive optical signals from the LRE **70,** to display pressure data therefrom, and/or transfer the pressure data to a computer using, for example, a serial interface. The optical detection unit **84** may further be adapted to provide a laser light source for reflection by the LRE **70**. The optical fibres **74** may reside in the sheath as such, or may be bundled together into the lumen of a flexible tubular housing **74** maintaining the individual fibres in intimate longitudinal contact so reducing the profile of the elongated member; the flexible tubular housing **74** is disposed within the sheath **14** as shown, for example, in **FIG. 8A**.

As explained later below, the proximal **20** end of the sheath body **14** is in sealed connection with a coupling **40** for an endoscope rinse port **250, 254,** which may be the rinse inlet port **254** or the rinse outlet port **250,** preferably the rinse inlet port **254**. The coupling **40** is configured to seal the proximal **20** region of the sheath **14** body with the relevant rinse port **250, 254** of the endoscope **200**. More in particular, it is configured to allow optical fibres **72** connecting the LRE **70** to exit the outer sheath **14,** while providing a separate fluidic connection to the relevant rinse port **250, 254** of the endoscope **200** hub **210**.

As a general guidance, maximum outer diameter, OD, of the sheath **14 (FIG. 8A),** particularly in the region that will pass through the relevant rinse lumen **232, 234** may be equal to or less than 0.3 mm, 0.4 mm, 0.5 mm, 0.6 mm, 0.7 mm, 0.8 mm, 0.9 mm, 1.0 mm, 1.1 mm, 1.2 mm, 1.3 mm, 1.4 mm, 1.5 mm or a value in the range between any two of the aforementioned values, preferably between 0.4 mm and 0.7 mm, 0.5 mm and 0.7 mm, most preferably 0.6 mm; the preferred values are particularly applicable when the inner diameter of the rinse inlet lumen or the rinse outlet lumen is 1.67 mm.

The dimensions of the luminal length, **LL (FIG. 3)** mentioned above in respect of the elongated member **10** apply equally to the sheath **14**.

The luminal length, **LL (FIG. 3)** of the sheath **14** between the coupling **40** and the distal open port **14** is preferably equal to or less than the length of the rinse lumen **234** in which the assembly **100** is deployed. The luminal length, **LL** may be fixed. Alternatively, the luminal length, **LL** may be adjustable, for instance, by employing the coupling **40** in sliding relation to the sheath **14**.

The wall of the sheath **14** may be formed from any suitable material such as polyethylene or polypropylene, polyimide, polyether ether ketone (PEEK).

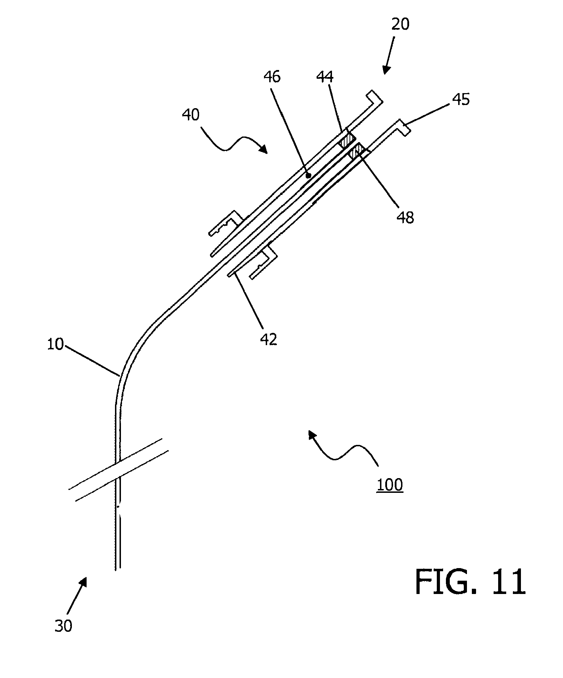

The assembly **100** is provided with a coupling **40** adapted for dismountable connection to the rinse port **250, 254** of the endoscope **200** hub **210**. The rinse port **250, 254** may be the rinse inlet port **254** or the rinse outlet port **250,** preferably the rinse inlet port **254**. The coupling **40** is positioned substantially in the proximal **20** half, or at the proximal end of the elongated member **10**. As exemplified in **FIG. 11,** the coupling **40** preferably has an essentially longitudinal shape, a proximal **20** and distal **30** end, and is provided with a fluid-tight internal chamber **46**. The coupling is disposed around the elongated member **10,** in a collar-like arrangement; the respective longitudinal axes may be aligned essentially co-axially.

At the distal end **30** of the coupling **40** is an open connector **42** in fluidic connection with the chamber **46,** adapted to engage with a rinse port **250, 254** of the endoscope hub **210**. The elongated member **10** is disposed through this open connector **42**. The connector **42** is preferably essentially tubular, hollow and threaded; more preferably it is a male Luer push connector in concentric alignment with an outer rotating female screw thread.

The coupling **40** is also provided with a proximal port **44**. The proximal port **44** is positioned toward the proximal **20** end of the coupling **40**. Preferably, a central axis of the port **44** is in co-axial alignment with a central axis of the connector **42**.

According to a preferred aspect of the invention, the body of the proximal **20** portion or end of the elongated member **10** is disposed through the proximal port **44**. This arrangement facilitates passage of the elongated member **10** through both the connector **42** and the port **44** without the requirement to bend or shape the elongated member **10.** The proximal port **44** is fluidically sealed around the outer body of the elongated member **10**. This may be achieved using, for example, a plug **48** of silicone or rubberized polymeric resin molded around the outer body of the elongated member **10.** *In situ* on the endoscope hub **210,** the chamber **46** of the coupling **40** is in sealed fluidic connection with the rinse inlet lumen **234.** Since the proximal end or part of the elongated member **10** passes through the proximal port **44** and the proximal port **44** is sealed there around, the chamber **46** is fluidically isolated from the proximal **20** end of the elongated member **10**. The proximal port **46** may be extended proximally **20** to include a connector **45** for dismountable attachment to an external device. The connector may be, for example, a Luer connector for dismountable attachment to a pressure gauge.

The embodiment shown in **FIG. 11** depicts a coupling **40** devoid of a side port, which is not required when the inlet port **254** on the hub **210** is already provided with a side port or valve allowing access to a rinse lumen **232, 234.**

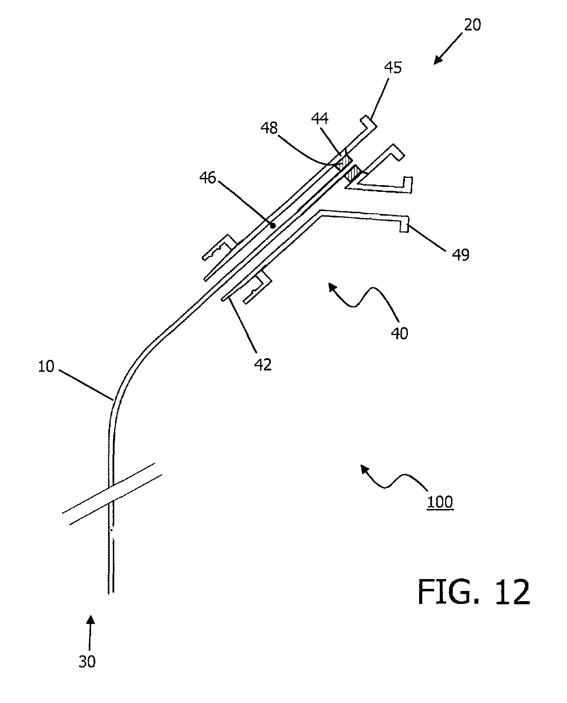

Alternatively, however, the coupling **40** may further be provided with a side port **49** in fluid connection with the chamber **46** as exemplified in **FIG. 12**. The side port **49** is positioned between the distal connector **42** and the proximal port **44**. *In situ* on the endoscope hub **210,** the chamber **46** of the coupling **40** is in sealed fluidic connection with a rinse lumen **232, 234** and the side port **49** allows access to the rinse lumen **232, 234,** permitting the introduction or escape of rinse fluid.

It is within the scope of the invention that the elongated member **10** passes either through the proximal port **44** as exemplified in **FIGs. 11** and **12** and as explained above, or through the side port **39**. Where it passes through and is sealed against the side port **39,** the proximal port **44,** rather than the side port **39** allows access to a rinse lumen **232, 234,** permitting the introduction or drainage of rinse fluid.

The coupling **40** may be provided in slidable or fixed relation to the elongated member **10**. Generally, but not necessarily, it will be in fixed relation when the elongated member **10** is a catheter **12** that can be trimmed from the distal end so as to change its luminal length **LL** according to the length of the relevant rinse lumen **232, 234** of the endoscope **200**. Generally, but not necessarily, it may be in sliding relation, when the pressure detecting body **50** is an electrical pressure transducer **16** and thus the possibility to truncate the elongated member **10,** formed of conducting wires, to match the length of the rinse lumen **232, 234** of the endoscope **200** cannot be realised. The coupling **40** may be provided in fixed relation by utilizing the sealing plug **48** of rubberized or silicone material to frictionally or adhesively engage with the outer wall of the elongated member **10**. The coupling may be provided in lockable, slidable relation by utilizing the plug **48** of rubberized or silicone material as a sliding seal that engages with a friction-reduced (*e.g.* Teflon coated) outer wall of the elongated member **10**. It may be locked by any means, such as a locking pin or screw.

Preferably, the coupling **40** is a standard three-branch, Y-shaped hub, two branches of the hub each provided with a female Luer push fitting around which a male thread is disposed, and one branch of the hub provided with a male Luer push-fitting about which a female rotating threaded collar is provided.

The pressure detecting body **50** is a structure located at the distal **30** end of pressure detecting body **50** onto which the pressure in the environment of the cavity is applied. Using either hydrostatic transmission (**FIG. 4**), or electrical transmission (**FIG. 6**), or optical transmission (**FIG. 8**), pressure in the tissue cavity applied to the pressure detecting body **50** is conveyed to the proximal end of the elongated member **10** where it can be read. In other words, the pressure detecting body **50** is configured to generate a conductible indication of ambient pressure, which may be conveyed *via*, for example, hydrostatic forces, electrical signals, or optical signals to the proximal end. The conductible indication of ambient pressure is in general terms a signal such as a level of hydrostatic pressure, an electrical or optical signal. The pressure detecting body is confined to the distal end of the elongated member **10**.

According to one embodiment, the pressure detecting body **50** comprises a distal port **14** (**FIG. 4**) **-** that is an opening in the shaft of the catheter - through which non-compressible fluid is displaced responsive to pressure in the cavity, which displacements are transmitted *via* a catheter **12** lumen to a pressure gauge connected to the proximal end of the catheter. The distal port **14** may be open or may be sealably covered with a flexible, expandable membrane. The distal port **14** is preferably, but not necessarily, located at the distal tip of the catheter **12** *i.e.* it is an end hole. Alternatively or in addition, it may be located on the side wall of the catheter but no more than 1 mm , 2 mm, 3 mm, 4 mm, 5 mm, 6 mm or 7 mm from the distal **30** tip of the catheter **12.** There may be one or more distal ports **14;** there are several ports, they will preferably be confined to the aforementioned distances from the distal **30** tip of the catheter **12.** The pressure detecting body **50** can be regarded as a portion of the catheter **12** located at its distal **30** end onto which the pressure in the environment of the cavity is applied, and is equalized with pressure within the lumen *via* the distal port **14.**

According to another embodiment, the pressure detecting body **50** may be an electrical pressure transducer **16 (FIG. 6)** that is a pressure sensing device that outputs an electrical signal responsive to pressure changes. Catheter tip pressure transducers are well known in the art, such as those used on the Codman Microsensor™ tip sensor (Johnson & Johnson Professional, Raynham, MA, USA). Typically, the transducer **16** has a transverse sectional profile which is the same as the elongated member, preferably cylindrical, though may alternatively be ovoid or polygonal. The transducer may comprise a piezoelectric transducer.

According to another embodiment, the pressure detecting body **50** may be a light-reflective element (LRE), such as a mirror, deformable on the application of pressure (**FIG. 8**). Light arriving at the LRE, through a fibre optic in the elongated tubular member is reflected by the LRE responsive to ambient pressure, and returned back along the elongated tubular member *via* a separate fibre optic. Distortions of the light-reflective element may cause a phase shift which is then measured in the optical detection unit. Typically, the LRE **70** has a transverse sectional profile which is the same as the elongated member, preferably cylindrical, though may alternatively be ovoid or polygonal.

It is within the scope of the invention that the elongated member **10** is provided as a further channel to the endoscope. In other words, an endoscope comprises an additional channel having the same properties as the elongated member **10,** in particular, the catheter, and the associated pressure detecting body **50.** The additional channel may not necessarily be disposed within the rinse inlet lumen **234;** it may be provided alongside. The endoscope is preferably single use; the single use endoscope avoids the need to sterilize the elongated member **10** which is technically difficult when it is a catheter **12** having a small diameter inner lumen.

One embodiment of the invention is continuous flow endoscope **200,** as described elsewhere herein, having an outer shaft that encloses a plurality of lumens, further comprising:

- an elongated member **10** having a proximal **20** and distal **30** end, disposed within the shaft,
- a pressure detecting body **50** at the distal **30** end of the elongated member **10,** configured to provide an indication of ambient pressure,
- said elongated member **10** configured to conduct the indication of ambient pressure to its proximal **20** end along the elongated member.

Another embodiment of the invention is a continuous flow endoscope as described above, where the elongated member **10** has one or more of the features defined elsewhere herein.

One embodiment of the invention is a kit comprising a continuous flow endoscope **200** as described herein, and a pressure detection assembly **100** as described herein. The luminal length, **LL (FIG. 3)** of elongated member **10** may be fixed so as to place the distal **30** tip of the pressure detecting body **50** flush or recessed with the distal **30** tip of the rinse inlet lumen **234** or the endoscope shaft **220** when the pressure detection assembly **100** is coupled to a rinse port **250, 254** of the endoscope hub **210.** The rinse port **250, 254** may be the rinse inlet port **254** or the rinse outlet port **250,** preferably the rinse inlet port **254.**

The elongated member **10** may be provided in the kit mounted or unmounted on the endoscope **200.** One embodiment of the invention is an endoscope as described herein, adapted with a pressure detection assembly **100** as described herein.

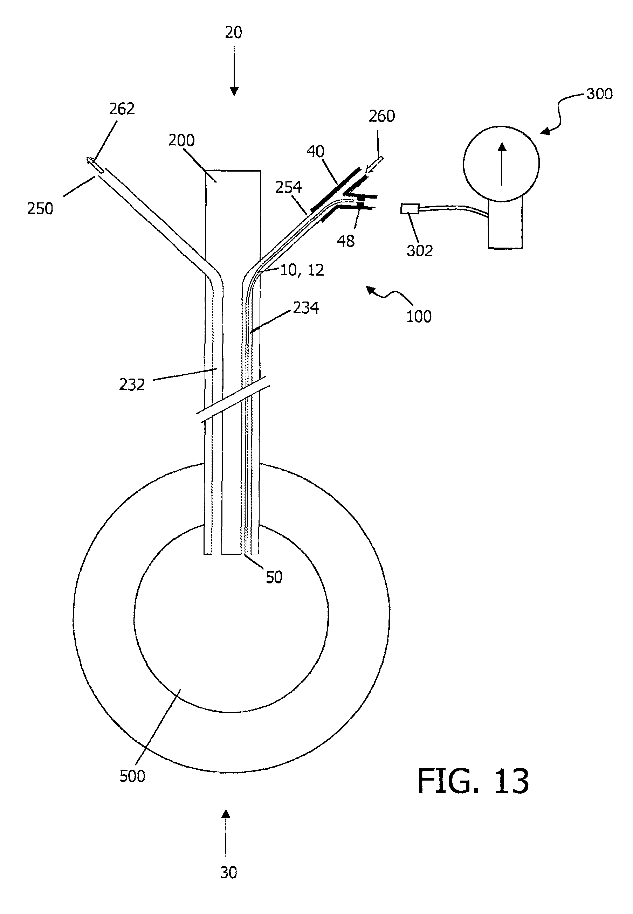

Shown in **FIG. 13,** is a pressure detection assembly **100** of the invention connected to an endoscope **200,** the endoscope having been inserted into a tissue cavity **500.** The elongated member **10,** a catheter **12,** is disposed within the rinse inlet lumen **234.** The distal **30** tip of the pressure detecting body **50** is flush with the distal **30** tip of the rinse inlet lumen **234** or endoscope shaft **200.** The coupling **40** mounted on the inlet port **254** fluidicly isolates the inlet port **254** from the proximal **20** end of the elongated member **10,** therefore, rinse medium provided **260** through a branch of coupling **40** does not enter the proximal end of the catheter **12.** The distal end of the catheter **12** can be connected to a pressure gauge **300.** Also depicted is the rinse outlet lumen **230** and associated proximal port **250** through which waste irrigation fluid is expelled **262.**

Another embodiment of the invention relates to a method for adapting a continuous flow endoscope **200** having a rinse inlet lumen **234** to provide an ambient pressure measurement capability. The method comprises the step of attaching the pressure detection assembly **100** of the present invention to the continuous flow endoscope according to the embodiments already described herein. In particular, it may comprise the steps:

(a) providing a pressure detection assembly **100** of the present invention,

(b) advancing the proximal end of the pressure detection assembly **100** through a rinse port **250, 254** of the endoscope **200,**

(c) engaging the coupling **40** with the rinse port **250, 254,** thereby adapting the continuous flow endoscope **200** to provide an ambient pressure measurement capability.

The rinse port **250, 254** may be the rinse inlet port **254** or the rinse outlet port **250,** preferably the rinse inlet port **254.**

The elongated member **10** is dimensioned to position the distal tip of the pressure detecting body **50** flush or recessed with respect to the distal tip of a rinse lumen **232, 234** or the endoscope shaft **200.** Where the elongated member **10** is a catheter, the lumen may be filled with an incompressible fluid, such as aqueous saline solution. Filling is effected by using, for example, a syringe, a saline bag, or fluid pump.

Another embodiment of the invention relates to a method for the measurement of ambient pressure within a tissue cavity being investigated with a continuous flow endoscope by the use of a transendoscopic pressure measurement probe. The transendoscopic pressure measurement probe is preferably comprised in a pressure detection assembly **100** of the present invention.

**EXAMPLE**

1. Materials and Methods

A custom-made model of a human head was used for the *in-vitro* measurements. The head was completely filled with 0.9% saline solution and sealed hermetically. A precoronal burr hole was made and closed with a rubber seal. A Caemaert endoscope (Richard Wolf, Knittlingen, Germany) was installed through the seal and fixated with a pneumatic holding device (Aesculap, Tuttlingen, Germany). The inflow- and outflow channels of the endoscope had an internal diameter of 1.67 mm and a length of 350 mm. A second burr hole was made and sealed with a rubber seal. A standard external ventricular drain with an internal diameter of 1.3 mm (Integra NeuroSciences, Plainsboro, NJ, USA) was positioned through the seal into the fluid-filled cavity. The rinsing system was installed in the standard manner for neuro-endoscopic procedures: 3-way stop cocks (Discofix®, B. Braun, Melsungen, Germany) were connected at the rinsing inlet and at the rinsing outlet for pressure measurement. Pressure transducers (PMSET 1DT-XX Becton Dickinson Critical Care Systems Pte Ltd, Singapore) were connected to the three-way stopcock and to the ventricular catheter *via* low compliance pressure tubing. All pressure transducers were flushed with saline, and zeroed at the level of the external acoustic meatus.

The irrigation system was installed as in routine clinical practice: a pressurised flush bag of saline was connected to the valve at the inflow of the endoscope *via* an infusion set with standard flow regulator. The bag was placed under a constant pressure of 300mmHg using a Ranger Pressure Infusion Systems (Arizant Inc., MN, USA). An IV infusion set (Intrafix Primeline I.S., B.Braun, Melsungen, Germany) was used as outflow tube. The luer-lock was connected to the 3-way stopcock at the rinsing outlet of the endoscope, and the opposite end was positioned at the level of the burr hole. For precise determination of the flow rate during pressure measurements, the effluent was collected into an accurate measuring glass for exactly 60 seconds.

All pressure transducers were connected to an S5 monitor (GE Health Care, Helsinki, Finland), which displayed the analogue pressure waveforms in real time, digitised the signals at a sampling frequency of 100 Hz, and transmitted them to a PC computer for electronic storage using S5 collect® software (GE Health Care, Helsinki, Finland).

Four separate experiments were performed. At the start of each experiment, the endoscope was introduced into the ventricular cavities, and a rinsing flow at "fast dripping speed" was initiated, as per routine clinical practice. After measurement of baseline pressures, the flow was increased in small steps, using the flow regulator, until a flow of 210 ml/min was reached. After each change in flow, an equilibration time was observed until a steady plateau pressure was reached. For each flow rate the plateau pressure was recorded. The rinsing fluid used was saline 0.9%.

2. Measurement 1

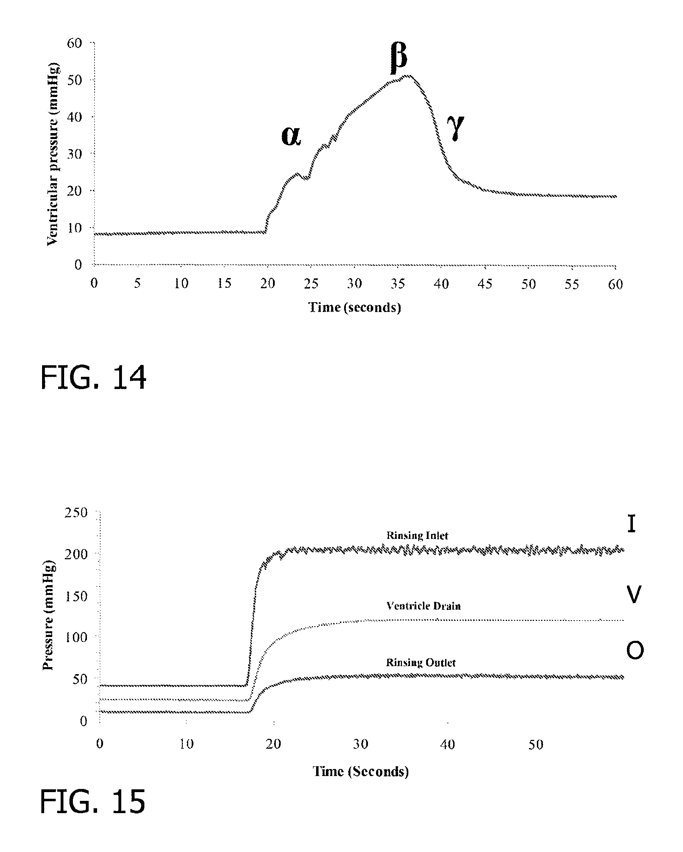

Pressure measurement was made according to the prior art i.e. the ventricular pressures were measured via the ventricular catheter and compared with the pressures measured at the rinsing inlet and rinsing outlet. The result of the measurement is shown in **FIG. 14.**

3. Measurement 2

In a second step, the equipment set-up was modified to enable pressure measure at the distal end of the lumen of the endoscope. A connecting piece (Rotating Male Hub Tuohy Borst with Sideport nr 80346, Qosina, Edgewood, New York, USA) was attached to the endoscope, and a Codman Microsensor™ tip sensor (Johnson & Johnson Professional, Raynham, MA, USA) was introduced through the rinse inlet lumen and advanced so that it was located 1mm proximal of the distal end of the endoscope. The tip sensor was also connected to the S5 monitor. The pressures it recorded were then compared with the pressure in the ventricle and at the rinsing outlet.

4. Measurement 3

The second protocol was repeated but instead of the Codman tip sensor, a Portex epidural catheter (Smiths Medical, NH, USA) was used. Before placement the distal 2.5 cm of the catheter, containing side holes, was removed to provide a catheter an end hole. The catheter was then slid through the inflow-channel until the tip was 1mm proximal to the distal end of the endoscope.

5. Measurement 4

The first measurement protocol was repeated but with a short Caemaert endoscope which also has a rinsing channel diameter of 1.67 mm, but a shaft length of 240 mm (as opposed to 350 mm in the standard instrument).

6. Data analysis

In the subsequent analysis, for each flow, the steady-state pressures at the different measuring points were graphically represented. The relationship between flow and pressure were determined by linear regression. The difference between the pressure in the ventricle - which is considered the gold standard - and the other pressure measurement sites was calculated for each flow rate.
The Reynolds number was calculated for each flow rate to evaluate whether laminar flow was likely. For each flow rate, at which laminar flow was likely (up to 180 ml/min), the measured pressure gradients were compared with pressure gradients predicted by the Hagen-Poiseuille equation [1]:

$$\Delta P = \frac{8\mu LQ}{\pi r^4}$$

[1]

Data were normally distributed and are presented as mean (SD).

7. Results

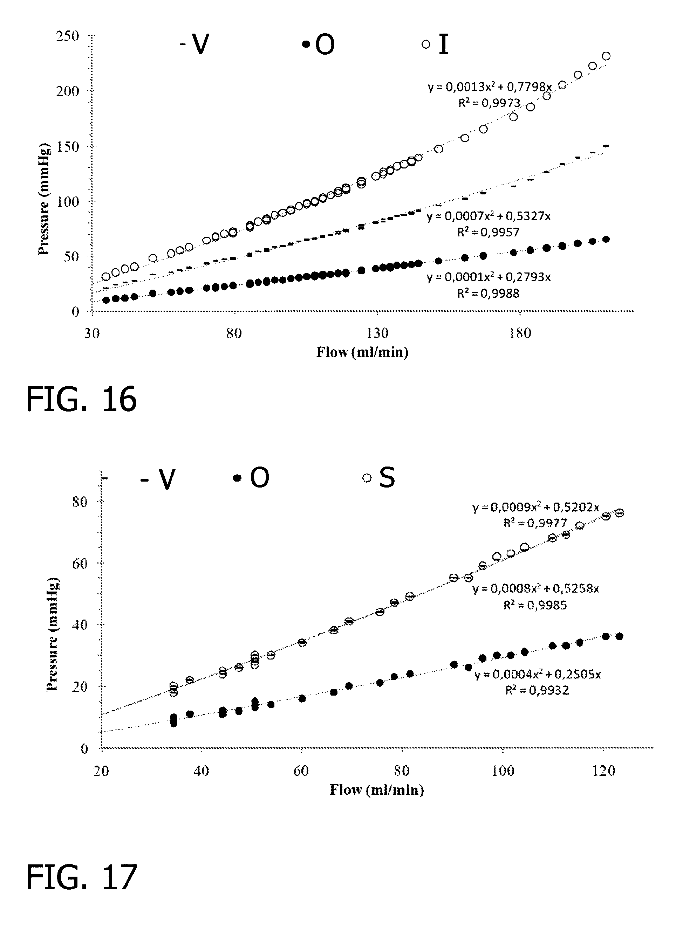

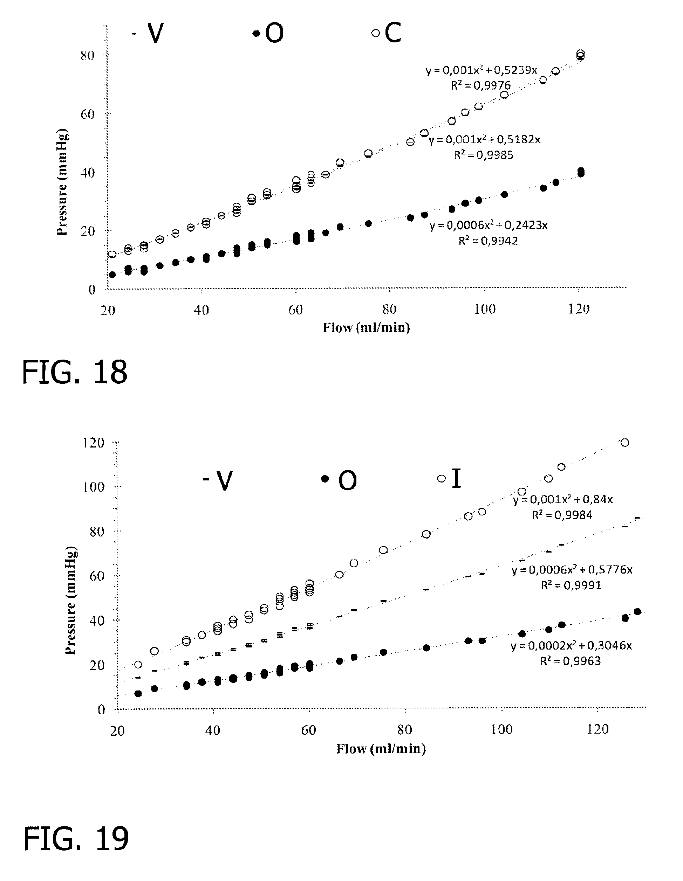

The evolution of the ventricular pressure during initiation of rinsing is shown in **FIG. 14.** Before the rinsing was started, a ventricular pressure of 8 mmHg was observed. At a flow of 85 ml/min, a peak pressure of 51 mmHg was reached, before the pressure stabilised at 18 mmHg.
FIG. 15 shows that when the rinsing flow is suddenly increased from a stable 40 ml/min to 185 ml/min, the ventricular pressure (V) increases from 25 to 122 mmHg, while the pressure at the inlet (I) increases from 42 to 223 mmHg and the pressure at the outlet (O) increases from 9 to 53 mmHg.
The pressure measured at the different points in relation to the flow is represented in **FIGs. 16 to 19.**
The pressure gradients between rinsing inlet (I), intraventricular (V), and rinsing outlet (O) related to the flow is shown (**FIG. 16**). At a flow of 42 ml/min the measured pressures are 38 mmHg, 26 mmHg and 12 mmHg respectively. At a flow of 135 ml/min the pressure increases to 136 mmHg, 89 mmHg and 42 mmHg respectively.
Both the Codman tip sensor **(FIG. 17)** and the epidural catheter measurement **(FIG. 18)** showed a maximal inaccuracy of -1 to 1 mmHg at any flow.
The short Caemaert Endoscope **(FIG. 19)** shows a similar evolution of the pressure gradient between the rinsing inlet (I), intraventricular (V), and rinsing outlet (O). At a flow of 24 ml/min the measured pressures are 20 mmHg, 14 mmHg and 7 mmHg, respectively. At a flow of 148 ml/min, the pressures increase to 146 mmHg, 99 mmHg, and 49 mmHg, respectively.
The Reynolds number, calculated for the dimension of the endoscope is 663 at a flow of 50ml/min up to 2650 at a flow of 200ml/min. At a flow of 61ml/min, the measured pressure gradient between rinsing inlet, intraventricular, and rinsing outlet was 18 mmHg and 19 mmHg respectively, while the theoretical pressure gradient, calculated by Poiseuille's equation is 17 mmHg. At a flow of 130 ml/min the measured pressure gradients are 31 mmHg and 31 mmHg; the calculated is 27 mmHg. At an extremely high flow of 210 ml/min the measured pressure gradients are 81 mmHg and 85 mmHg, while the calculated gradient is 57 mmHg.
After initiation of rinsing (flow 30 ml/min) in the model, only a transient period of intracranial hypertension was observed. The evolution of ventricular pressure changes during this period shows four phases **(FIG. 14).** During the first phase, pressures rise as the endoscope and the tubings fill with rinsing fluid **(FIG. 14, α)**, until reaching a peak of 51 mmHg **(FIG. 14, β)**. After the onset of the siphoning effect of the outflow tube, the ventricular pressure declines **(FIG. 14, γ)**, until the ventricular pressure settles at 18 mmHg, when the siphoning effect is balanced by the hydrostatic pressure in the outflow tube. If the distal end of the outflow tube is obstructed, absent or at an incorrect level, a continuously elevated ICP will be induced by the hydrostatic pressure in the outflow channel. The total ICP will be the sum of the hydrostatic pressure and the pressure buildup caused by impedance in the outflow channel. Conversely if the distal end of the outflow tube is located too low, the siphoning effect will cause a collapse of the ventricles. Increasing the rinsing flow results in a considerable increase in the pressure at all measuring points. In measurement 1, there were significant differences in pressure readings at the different locations. Monitoring at the rinsing inlet overestimated the ventricular pressure by 12 mmHg at 42 ml/min, and by 81 mmHg at 210 ml/min. On the other hand, monitoring at the rinsing outlet underestimated the ventricular pressure by 14 mmHg at 42 ml/min and by 85 mmHg at 210 ml/min. Similar differences were found with the short endoscope - an overestimation of ~41 mmHg and an underestimation of ~42 mmHg at the inlet and outlet ports at flow rates of 128 ml/min. This pressure difference is caused by the dynamic resistance in the rinsing channel, and correlates well with the pressure gradients predicted by the Hagen-Poiseuille law (difference of 1-2 mmHg at 61 ml/min increasing to 7-8 mmHg at 130 ml/min).

Transendoscopic monitoring of the pressure at the distal tip of the endoscope using an electronic Codman tip sensor provided a very accurate assessment of the ventricular pressure (and thus of the ICP). Of course, the application of an extra monitoring device and the use of a disposable electronic tip sensor will introduce some practical and financial considerations. In order to find a cheaper and more practical method of transendoscopic pressure monitoring, we replaced the tip sensor with a fluid-filled epidural anaesthesia catheter connected to a standard pressure transducer outside of the head. The tip of the catheter was placed at the same location as the tip sensor (1 mm proximal to the distal end of the endoscope). When intact epidural catheters are used, a systematic overestimation of the ventricular pressure occurs, because they have lateral side holes 7, 11 and 15 mm from the distal end, and thus the measured pressure reflects the pressure 15 mm proximal to the distal end of the endoscope. In the current study measurements were thus performed with a modified catheter containing only an end hole, and with this catheter the transendoscopic pressure measurements compared highly favourably with ventricular pressure measurements (maximal error of ± 1 mmHg).

Because the induced intracranial hypertension only becomes clinically relevant at faster rinsing flow rates - above 50ml/min - and the rinsing flow is relatively stable, the compliance of the intracranial system is of minimal influence on the observed pressure values. Based on the Monro-Kellie hypothesis - that with an intact skull, the sum of the volumes of the brain, the CSF and the intracranial blood is constant - the capacity for expansion of the intraventricular volume during fast rinsing flow rates is limited to the intracranial blood volume. During gradual flow rate increases, the induced blood volume displacement caused by changes in rinsing pressure is minimal compared to rinsing volumes. This is confirmed by our observation that after adjustment of the rinsing speed, the pressure-waveform stabilizes almost immediately. Nevertheless, when the pressure is increased rapidly and severely **(FIG. 14)**, it takes several seconds before stable pressure readings are observed.

The findings of this laboratory-based assessment suggest that clinically significant pressure gradients across the endoscope are generated during rinsing. These gradients are generated by dynamic resistances in the rinsing channels (Poiseuille law). Measurement at the rinsing inlet gives a severe overestimation of the true ICP (up to 50 mmHg), and if clinicians were to respond to these pressures, this would unnecessarily impede the rinsing efforts of the surgeon. Measurement at the outflow point gives a systematic severe underestimation of the true ICP (up to 50 mmHg), which would delay crucial intervention. Transendoscopic measurement of the pressure at the distal end of the endoscope accurately reflects the static ventricular pressure. There was no significant difference in the pressure measured at the tip of the endoscope using a Codman tip sensor (pressure transducer) and an epidural catheter.

**Claims**

1. A pressure detection assembly (100) adapted for use with a continuous flow endoscope (200) provided with a rinse lumen (232, 234) connected to a rinse port (250, 254) said assembly (100) comprising:

- an elongated member (10) having a proximal (20) and distal (30) end,
- a pressure detecting body (50) at the distal (30) end of the elongated member (10), configured to provide an indication of ambient pressure,
- a coupling (40) disposed over the elongated member (10), configured for dismountable attachment to the proximal rinse port (250, 254) of the endoscope (200),
- said elongated member (10) configured to conduct the indication of ambient pressure to its proximal (20) end,
- said elongated member (10) further configured for advancement through the rinse lumen (232, 234) and to maintain the flow functioning of the rinse lumen, and
- said fluidic coupling (40) further configured to isolate fluidicly the proximal (20) tip of the elongated member (10) from the rinse port (250, 254) of the endoscope (200) wherein:
- the elongated member (10) is a single lumen (15) catheter (12),
- the pressure detecting body (50) comprises the distal (30) tip of the catheter incorporating an open port (14) in fluidic connection with the catheter lumen (15), and
- the indication of ambient pressure is conducted by the catheter (12) using hydrostatic force.

2. A pressure detection assembly (100) according to claim 1, wherein the continuous flow endoscope (200) is provided with a rinse inlet lumen (234) connected to a rinse inlet port (254), and

- the rinse port to which the coupling (40) is configured for dismountable attachment is the rinse inlet port (254),
- the rinse lumen through which the elongated member (10) is configured for advancement through is the rinse inlet lumen (234) and maintains the flow functioning of the rinse inlet lumen (234), and
- the rise port from which the fluidic coupling (40) is configured to isolate fluidicly the proximal (20) tip of the elongated member (10) from is the rinse inlet port (254).

3. Assembly (100) according to any of claims 1 to 3, wherein the distal tip (30) of the elongated member (10) is configured for locating flush or recessed to the distal (30) tip of the rinse lumen (232, 234).

4. Assembly (100) according to claim 3, wherein the distal tip (30) of the elongated member (10) is configured for locating at a distance of 0 mm, 0.1 mm, 0.2 mm, 0.4 mm, 0.6 mm, 1 mm, 2 mm, 3 mm, 4 mm, 6 mm, 8 mm, or 10 mm proximal to the distal tip of the rinse inlet lumen (234) or endoscope shaft (220).

5. Assembly (100) according to any of claims 1 to 4, wherein the coupling (40) is configured to separate fluid access to the rinse lumen (232, 234) from fluid access to the catheter (12) lumen (15).

6. Assembly (100) according to any of claims 1 to 5, wherein the coupling (40) comprises a Luer lock fitting.

7. Assembly (100) according to any of claims 1 to 6, wherein the diameter of the catheter (10, 12) is between 0.3 mm and 0.9 mm, preferably between 0.4 mm and 0.7 mm.

8. Assembly (100) according to any of claims 1 to 7, wherein the length of the catheter (10, 12) between the distal tip and the coupling is between 10 cm and 35 cm, preferably between 14 cm to 24 cm in length.

9. Assembly (100) according to any of claims 1 to 8, wherein the flow functioning of the rinse lumen is maintained when the fluid flow through the rinse lumen (234) at constant pressure is reduced by an amount equal to or less than 60 % when the elongated member (10) is advanced therethrough.

10. A kit comprising:

- an assembly (100) as defined in any of the previous claims, and
- a continuous flow endoscope (200).

11. A method for adapting a continuous flow endoscope (200) with a rinse lumen (232, 234) connected to a rinse port (250, 254) to provide an ambient pressure measurement capability comprising the steps:

(a) providing a pressure detection assembly (100) as defined in any of claims 1 to 9,
(b) advancing the proximal end of the pressure detection assembly (100) through the rinse inlet port (254) of the endoscope (200), and
(c) engaging the coupling (40) with the rinse port (250, 254), thereby adapting the continuous flow endoscope (200) to provide an ambient pressure measurement capability.

12. A method for according to claim 11, wherein the rinse lumen (232, 234) is a rinse inlet lumen (234), and the rinse port (250, 254) is the rinse inlet port (254).

13. A method of preparing an assembly as defined to any of claims 1 to 9, comprising the step of attaching the fluidic coupling (40) to the shaft of the catheter (12) so as to allow separate fluid access to the rinse lumen (232, 234) and the catheter (12) lumen (15).

**Patentansprüche**

1. Druckdetektionsanordnung (100), die dafür ausgelegt ist, mit einem Endoskop (200) mit ununterbrochener Strömung, das mit einem Spülungslumen (232, 234) versehen ist, das mit einem Spülungsanschluss (250, 254) verbunden ist, verwendet zu werden, wobei die Anordnung (100) Folgendes umfasst:

   - ein lang gestrecktes Element (10) mit einem proximalen Ende (20) und einem distalen Ende (30),
   - einen Druckdetektionskörper (50) am distalen Ende (30) des lang gestreckten Elements (10), der konfiguriert ist, eine Angabe des Umgebungsdrucks bereitzustellen,
   - eine Kopplung (40), die über dem lang gestreckten Element (10) angeordnet ist und konfiguriert ist, an dem proximalen Spülungsanschluss (250, 254) des Endoskops (200) abnehmbar befestigt zu werden,
   - wobei das lang gestreckte Element (10) konfiguriert ist, die Angabe des Umgebungsdrucks zu seinem proximalen Ende (20) zu leiten,
   - wobei das lang gestreckte Element (10) ferner konfiguriert ist, sich durch das Spülungslumen (232, 234) vorwärts zu bewegen und die Strömungsfunktion des Spülungslumens aufrecht zu erhalten, und
   - wobei die fluidtechnische Kopplung (40) ferner konfiguriert ist, die proximale Spitze (20) des lang gestreckten Elements (10) von dem Spülungsanschluss (250, 254) des Endoskops (200) fluidtechnisch zu isolieren, wobei:
   - das lang gestreckte Element (10) ein Katheter (12) mit einzelnem Lumen (15) ist,
   - der Druckdetektionskörper (50) eine distale Spitze (30) des Katheters umfasst, der einen offenen Anschluss (14) in fluidtechnischer Verbindung mit dem Katheterlumen (15) enthält, und
   - die Angabe des Umgebungsdrucks durch den Katheter (12) unter Verwendung einer hydrostatischen Kraft geleitet wird.

2. Druckdetektionsanordnung (100) nach Anspruch 1, wobei das Endoskop (200) mit ununterbrochener Strömung mit einem Spülungseinlasslumen (234) versehen ist, das mit einem Spülungseinlassanschluss (254) verbunden ist, und

   - der Spülungsanschluss, in Bezug auf den die Kopplung (40) für eine abnehmbare Befestigung konfiguriert ist, der Spülungseinlassanschluss (254) ist,
   - das Spülungslumen, in Bezug auf das das lang gestreckte Element (10) für eine Vorwärtsbewegung konfiguriert ist, das Spülungseinlasslumen (234) ist und die Strömungsfunktion des Spülungseinlasslumens (234) aufrecht erhält, und
   - der Spülungsanschluss, in Bezug auf den die fluidtechnische Kopplung (40) konfiguriert ist, hiervon die proximale Spitze (20) des lang gestreckten Elements (10) fluidtechnisch zu isolieren, der Spülungseinlassanschluss (254) ist.

3. Anordnung (100) nach einem der Ansprüche 1 bis 3, wobei die distale Spitze (30) des lang gestreckten Elements (10) konfiguriert ist, bündig mit oder zurückversetzt zu der distalen Spitze (30) des Spülungslumens (232, 234) angeordnet zu werden.

4. Anordnung (100) nach Anspruch 3, wobei die distale Spitze (30) des lang gestreckten Elements (10) konfiguriert ist, in einem Abstand von 0 mm, 0,1 mm, 0, 2 mm, 0 , 4 mm, 0 , 6 mm, 1 mm, 2 mm, 3 mm, 4 mm, 6 mm, 8 mm oder 10 mm proximal zu der distalen Spitze des Spülungseinlasslumens (234) oder des Endoskopschafts (220) angeordnet zu werden.

5. Anordnung (100) nach einem der Ansprüche 1 bis 4, wobei die Kopplung (40) konfiguriert ist, den Fluidzugang zu dem Spülungslumen (232, 234) von einem Fluidzugang zu dem Lumen (15) des Katheters (12) zu trennen.

6. Anordnung (100) nach einem der Ansprüche 1 bis 5, wobei die Kopplung (40) eine Luer-Rohrverriegelungsverbindung umfasst.

7. Anordnung (100) nach einem der Ansprüche 1 bis 6, wobei der Durchmesser des Katheters (10, 12) im Bereich von 0,3 mm bis 0,9 mm und vorzugsweise im Bereich von 0,4 mm bis 0,7 mm liegt.

8. Anordnung (100) nach einem der Ansprüche 1 bis 7, wobei die Länge des Katheters (10, 12) zwischen der distalen Spitze und der Kopplung in Längsrichtung im Bereich von 10 cm bis 35 cm, vorzugsweise im Bereich von 14 cm bis 24 cm liegt.

9. Anordnung (100) nach einem der Ansprüche 1 bis 8, wobei die Strömungsfunktion des Spülungslumens aufrecht erhalten wird, wenn die Fluidströmung durch das Spülungslumen (234) mit konstantem Druck um einen Betrag reduziert wird, der gleich oder kleiner als 60 % ist, wenn das lang gestreckte Element (10) vorwärts hindurchbewegt wird.

10. Baueinheit, die folgendes umfasst:

- eine Anordnung (100) nach einem der vorhergehenden Ansprüche und
- ein Endoskop (200) mit ununterbrochener Strömung.

11. Verfahren, um ein Endoskop (200) mit ununterbrochener Strömung, das ein Spülungslumen (232, 234) besitzt, das mit einem Spülungsanschluss (250, 254) verbunden ist, dafür auszulegen, dass es die Fähigkeit einer Umgebungsdruckmessung bereitstellt, das die folgenden Schritte umfasst:

(a) Bereitstellen einer Druckdetektionsanordnung (100) nach einem der Ansprüche 1 bis 9,
(b) Vorwärtsbewegen des proximalen Endes der Druckdetektionsanordnung (100) durch den Spülungseinlassanschluss (254) des Endoskops (200) und
(c) Herstellen eines Eingriffs zwischen der Kopplung (40) und dem Spülungsanschluss (250, 254), um dadurch das Endoskop (200) mit ununterbrochener Strömung geeignet zu machen, die Fähigkeit zu einer Umgebungsdruckmessung bereitzustellen.

12. Verfahren nach Anspruch 11, wobei das Spülungslumen (232, 234) ein Spülungseinlasslumen (234) ist und der Spülungsanschluss (250, 254) der Spülungseinlassanschluss (254) ist.

13. Verfahren zum Vorbereiten einer Anordnung nach einem der Ansprüche 1 bis 9, das den Schritt des Befestigens der fluidtechnischen Kopplung (40) mit dem Schaft des Katheters (12) umfasst, um einen getrennten Fluidzugang zu dem Spülungslumen (232, 234) und dem Lumen (15) des Katheters (12) zu ermöglichen.

## Revendications

1. Ensemble de détection de pression (100) prévu pour être utilisé avec un endoscope à flux continu (200) pourvu d'une lumière de rinçage (232, 234) connectée par un orifice de rinçage (250, 254), ledit ensemble (100) comprenant :

- un organe allongé (10), ayant une extrémité proximale (20) et une extrémité distale (30),
- un corps de détection de pression (50) au niveau de l'extrémité distale (30) de l'organe allongé (10), configuré pour fournir une indication de la pression ambiante,
- un raccord (40) disposée par-dessus l'organe allongé (10), configuré de manière à pouvoir être attaché de manière amovible à l'orifice de rinçage proximal (250, 254) de l'endoscope (200),
- ledit organe allongé (10) étant configuré pour conduire l'indication de pression ambiante jusqu'à son extrémité proximale (20),
- ledit organe allongé (10) étant en outre configuré pour avancer à travers la lumière de rinçage (232, 234) et pour maintenir le fonctionnement d'écoulement de la lumière de rinçage, et
- ledit raccord fluidique (40) étant en outre configuré pour isoler fluidiquement la pointe proximale (20) de l'organe allongé (10) de l'orifice de rinçage (250, 254) de l'endoscope (200), dans lequel :
- l'organe allongé (10) est un cathéter (12) à lumière unique (15),
- le corps de détection de pression (50) comprend la pointe distale (30) du cathéter incorporant un orifice ouvert (14) en liaison fluidique avec la lumière de cathéter (15), et
- l'indication de pression ambiante est conduite par le cathéter (12) en utilisant une force hydrostatique.

2. Ensemble de détection de pression (100) selon la revendication 1, dans lequel l'endoscope à flux continu (200) est pourvu d'une lumière d'entrée de rinçage (234) connectée à un orifice d'entrée de rinçage (254), et

- l'orifice de rinçage auquel le raccord (40) est prévu pour être attaché de manière amovible est l'orifice d'entrée de rinçage (254),
- la lumière de rinçage à travers laquelle l'organe allongé (10) est prévu pour avancer à travers est la lumière d'entrée de rinçage (234) et maintient le fonctionnement d'écoulement de la lumière d'entrée de rinçage (234), et
- l'orifice de rinçage duquel le raccord fluidique (40) est prévu pour isoler fluidiquement la pointe proximale (20) de l'organe allongé (10) est l'orifice d'entrée de rinçage (254).

3. Ensemble (100) selon l'une quelconque des revendications 1 à 3, dans lequel la pointe distale (30) de l'organe allongé (10) est configurée pour se positionner en affleurement ou en retrait par rapport à la pointe distale (30) de la lumière de rinçage (232, 234).

4. Ensemble (100) selon la revendication 3, dans lequel la pointe distale (30) de l'organe allongé (10) est configurée pour se positionner à une distance de 0 mm, 0,1 mm, 0,2 mm, 0,4 mm, 0,6 mm, 1 mm, 2 mm, 3 mm, 4 mm, 6 mm, 8 mm, ou 10 mm à proximité de la pointe distale de la lumière d'entrée de rinçage (234) ou de la tige de l'endoscope (220).

5. Ensemble (100) selon l'une quelconque des revendications 1 à 4, dans lequel le raccord (40) est configuré pour séparer l' accès de fluide à la lumière de rinçage (232, 234) de l'accès de fluide à la lumière (15) du cathéter (12).

6. Ensemble (100) selon l'une quelconque des revendications 1 à 5, dans lequel le raccord (40) comprend un raccord à connecteur de verrouillage Luer.

7. Ensemble (100) selon l'une quelconque des revendications 1 à 6, dans lequel le diamètre du cathéter (10, 12) est compris entre 0,3 mm et 0,9 mm, de préférence entre 0,4 mm et 0,7 mm.

8. Ensemble (100) selon l'une quelconque des revendications 1 à 7, dans lequel la longueur du cathéter (10, 12) entre la pointe distale et le raccord est comprise entre 10 cm et 35 cm, de préférence entre 14 cm et 24 cm de longueur.

9. Ensemble (100) selon l'une quelconque des revendications 1 à 8, dans lequel le fonctionnement d'écoulement de la lumière de rinçage est maintenu lorsque l'écoulement de fluide à travers la lumière de rinçage (234) à pression constante est réduit d'une quantité inférieure ou égale à 60 % lorsque l'organe allongé (10) est avancé à travers elle.

10. Kit comprenant :

   - un ensemble (100) selon l'une quelconque des revendications précédentes, et
   - un endoscope (200) à flux continu.

11. Procédé pour adapter un endoscope (200) à flux continu comprenant une lumière de rinçage (232, 234) connectée à un orifice de rinçage (250, 254) pour fournir une capacité de mesure de pression ambiante comprenant les étapes suivantes :

   (a) fournir un ensemble de détection de pression (100) selon l'une quelconque des revendications 1 à 9,
   (b) faire avancer l'extrémité proximale de l'ensemble de détection de pression (100) à travers l'orifice d'entrée de rinçage (254) de l'endoscope (200), et
   (c) mettre en prise le raccord (40) avec l'orifice de rinçage (250, 254), pour ainsi adapter l'endoscope (200) à flux continu de manière à fournir une capacité de mesure de pression ambiante.

12. Procédé selon la revendication 11, dans lequel la lumière de rinçage (232, 234) est une lumière d'entrée de rinçage (234), et l'orifice de rinçage (250, 254) est l'orifice d'entrée de rinçage (254).

13. Procédé de préparation d'un ensemble selon l'une quelconque des revendications 1 à 9, comprenant l'étape consistant à attacher le raccord fluidique (40) à la tige du cathéter (12) de manière à permettre un accès fluidique séparé à la lumière de rinçage (232, 234) et à la lumière (15) du cathéter (12).

Q=135ml/min

64  63    55       60  61

65

P=42mmHg              P=136mmHg    300

234

210

230

200

68

P=89mmHg

P=89mmHg

VENTRICLE

BRAIN
PARENCHYMA

21

22

PRIOR ART

FIG. 1

EP 2 598 015 B1

FIG. 2

FIG. 3

FIG. 3A

EP 2 598 015 B1

FIG. 4

FIG. 5

FIG. 4A

FIG. 6A

FIG. 6

FIG. 7

FIG. 8

FIG. 8A

FIG. 9

EP 2 598 015 B1

FIG. 10

FIG. 11

FIG. 12

FIG. 13

FIG. 14

FIG. 15

FIG. 16

FIG. 17

FIG. 18

FIG. 19

**EP 2 598 015 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

*   US 2009182201 A